# EUROPEAN PATENT APPLICATION

(11) **EP 1 201 765 A2**
(43) Date of publication of application: **02.05.2002**
(21) Application number: 01124604.8
(22) Date of filing: 15.10.2001
(51) Int. Cl.: C12Q 1/48, C07K 16/00, A61P 31/12, A61K 39/395, A61K 48/00, C12Q 1/68

(54) **Cellular kinases involved in cytomegalovirus infection and their inhibition**

(30) Priority: 16.10.2000 US 240750 P
(71) Applicant: Axxima Pharmaceuticals Aktiengesellschaft, 82152 Martinsried (DE)
(72) Inventor: Schubart, Daniel, 79576 Weil am Rhein (DE); Habenberger, Peter, 81373 Munich (DE); Stein-Gerlach, Matthias, 81475 Munich (DE); Bevec, Dorian, 82110 Germering (DE)
(74) Representative: Leidescher, Thomas

(57) **Abstract**

The role of certain cellular kinases active during Human Cytomegalovirus infection is disclosed. These cellular kinases are useful to detect HCMV infection, and can be used to screen for cellular kinase inhibitors. Cellular kinases inhibitors, which effectively downregulate these key cellular components, serve as effective therapeutics against HCMV infection.

SEQUENCE LISTING

<110> Axxima Pharmaceuticals AG

<120> CELLULAR KINASES INVOLVED IN CYTOMEGALOVIRUS INFECTION
      AND THEIR INHIBITION

<130> PA012EP

<140>
<141>

<160> 2

<170> PatentIn Ver. 2.1

<210> 1
<211> 2501
<212> DNA
<213> Homo sapiens

<220>
<221> CDS
<222> (225)..(1847)

<400> 1
ggcaccagtc tctagaaaag aagtcagctc tggttcggag aagcagcggc tggcgtgggc 60

catccgggga atgggcgccc tcgtgaccta gtgttgcggg gcaaaaaggg tcttgccggc 120

ctcgctcgtg caggggcgta tctgggcgcc tgagcgcgca gtgggagcct tgggagccgc 180

cgcagcaggg ggcacacccg gaaccggcct gagcgcccgg gacc atg aac ggg gag 236
                                                 Met Asn Gly Glu
                                                   1

gcc atc tgc agc gcc ctg ccc acc att ccc tac cac aaa ctc gcc gac 284
Ala Ile Cys Ser Ala Leu Pro Thr Ile Pro Tyr His Lys Leu Ala Asp
  5                  10                  15                  20

ctg cgc tac ctg agc cgc ggc gcc tct ggc act gtg tcg tcc gcc cgc 332
Leu Arg Tyr Leu Ser Arg Gly Ala Ser Gly Thr Val Ser Ser Ala Arg
                 25                  30                  35

cac gca gac tgg cgc gtc cag gtg gcc gtg aag cac ctg cac atc cac 380
His Ala Asp Trp Arg Val Gln Val Ala Val Lys His Leu His Ile His
             40                  45                  50

act ccg ctg ctc gac agt gaa aga aag gat gtc tta aga gaa gct gaa 428
Thr Pro Leu Leu Asp Ser Glu Arg Lys Asp Val Leu Arg Glu Ala Glu
         55                  60                  65

att tta cac aaa gct aga ttt agt tac att ctt cca att ttg gga att 476
Ile Leu His Lys Ala Arg Phe Ser Tyr Ile Leu Pro Ile Leu Gly Ile
     70                  75                  80

tgc aat gag cct gaa ttt ttg gga ata gtt act gaa tac atg cca aat 524
Cys Asn Glu Pro Glu Phe Leu Gly Ile Val Thr Glu Tyr Met Pro Asn
 85                  90                  95                 100

gga tca tta aat gaa ctc cta cat agg aaa act gaa tat cct gat gtt 572
Gly Ser Leu Asn Glu Leu Leu His Arg Lys Thr Glu Tyr Pro Asp Val
                105                 110                 115

gct tgg cca ttg aga ttt cgc atc ctg cat gaa att gcc ctt ggt gta 620
Ala Trp Pro Leu Arg Phe Arg Ile Leu His Glu Ile Ala Leu Gly Val
            120                 125                 130

aat tac ctg cac aat atg act cct cct tta ctt cat cat gac ttg aag 668
Asn Tyr Leu His Asn Met Thr Pro Pro Leu Leu His His Asp Leu Lys
        135                 140                 145

act cag aat atc tta ttg gac aat gaa ttt cat gtt aag att gca gat 716
Thr Gln Asn Ile Leu Leu Asp Asn Glu Phe His Val Lys Ile Ala Asp
    150                 155                 160

ttt ggt tta tca aag tgg cgc atg atg tcc ctc tca cag tca cga agt 764
Phe Gly Leu Ser Lys Trp Arg Met Met Ser Leu Ser Gln Ser Arg Ser
165                 170                 175                 180

agc aaa tct gca cca gaa gga ggg aca att atc tat atg cca cct gaa 812
Ser Lys Ser Ala Pro Glu Gly Gly Thr Ile Ile Tyr Met Pro Pro Glu
                185                 190                 195

aac tat gaa cct gga caa aaa tca agg gcc agt atc aag cac gat ata 860
Asn Tyr Glu Pro Gly Gln Lys Ser Arg Ala Ser Ile Lys His Asp Ile
            200                 205                 210

tat agc tat gca gtt atc aca tgg gaa gtg tta tcc aga aaa cag cct 908
Tyr Ser Tyr Ala Val Ile Thr Trp Glu Val Leu Ser Arg Lys Gln Pro
        215                 220                 225

ttt gaa gat gtc acc aat cct ttg cag ata atg tat agt gtg tca caa 956
Phe Glu Asp Val Thr Asn Pro Leu Gln Ile Met Tyr Ser Val Ser Gln
    230                 235                 240

gga cat cga cct gtt att aat gaa gaa agt ttg cca tat gat ata cct 1004
Gly His Arg Pro Val Ile Asn Glu Glu Ser Leu Pro Tyr Asp Ile Pro
245                 250                 255                 260

cac cga gca cgt atg atc tct cta ata gaa agt gga tgg gca caa aat 1052
His Arg Ala Arg Met Ile Ser Leu Ile Glu Ser Gly Trp Ala Gln Asn
                265                 270                 275

cca gat gaa aga cca tct ttc tta aaa tgt tta ata gaa ctt gaa cca 1100
Pro Asp Glu Arg Pro Ser Phe Leu Lys Cys Leu Ile Glu Leu Glu Pro
            280                 285                 290

gtt ttg aga aca ttt gaa gag ata act ttt ctt gaa gct gtt att cag 1148
Val Leu Arg Thr Phe Glu Glu Ile Thr Phe Leu Glu Ala Val Ile Gln
        295                 300                 305

cta aag aaa aca aag tta cag agt gtt tca agt gcc att cac cta tgt 1196
Leu Lys Lys Thr Lys Leu Gln Ser Val Ser Ser Ala Ile His Leu Cys
    310                 315                 320

gac aag aag aaa atg gaa tta tct ctg aac ata cct gta aat cat ggt 1244
Asp Lys Lys Lys Met Glu Leu Ser Leu Asn Ile Pro Val Asn His Gly
325                 330                 335                 340

cca caa gag gaa tca tgt gga tcc tct cag ctc cat gaa aat agt ggt 1292
Pro Gln Glu Glu Ser Cys Gly Ser Ser Gln Leu His Glu Asn Ser Gly
                345                 350                 355

tct cct gaa act tca agg tcc ctg cca gct cct caa gac aat gat ttt 1340
Ser Pro Glu Thr Ser Arg Ser Leu Pro Ala Pro Gln Asp Asn Asp Phe
            360                 365                 370

tta tct aga aaa gct caa gac tgt tat ttt atg aag ctg cat cac tgt 1388
Leu Ser Arg Lys Ala Gln Asp Cys Tyr Phe Met Lys Leu His His Cys
        375                 380                 385

cct gga aat cac agt tgg gat agc acc att tct ggt tct caa agg gct 1436
Pro Gly Asn His Ser Trp Asp Ser Thr Ile Ser Gly Ser Gln Arg Ala
    390                 395                 400

gca ttc tgt gat cac aag acc act cca tgc tct tca gca ata ata aat 1484
Ala Phe Cys Asp His Lys Thr Thr Pro Cys Ser Ser Ala Ile Ile Asn
405                 410                 415                 420

cca ctc tca act gca gga aac tca gaa cgt ctg cag cct ggt ata gcc 1532
Pro Leu Ser Thr Ala Gly Asn Ser Glu Arg Leu Gln Pro Gly Ile Ala
                425                 430                 435

cag cag tgg atc cag agc aaa agg gaa gac att gtg aac caa atg aca 1580
Gln Gln Trp Ile Gln Ser Lys Arg Glu Asp Ile Val Asn Gln Met Thr
            440                 445                 450

gaa gcc tgc ctt aac cag tcg cta gat gcc ctt ctg tcc agg gac ttg 1628
Glu Ala Cys Leu Asn Gln Ser Leu Asp Ala Leu Leu Ser Arg Asp Leu
        455                 460                 465

atc atg aaa gag gac tat gaa ctt gtt agt acc aag cct aca agg acc 1676
Ile Met Lys Glu Asp Tyr Glu Leu Val Ser Thr Lys Pro Thr Arg Thr
    470                 475                 480

tca aaa gtc aga caa tta cta gac act act gac atc caa gga gaa gaa 1724
Ser Lys Val Arg Gln Leu Leu Asp Thr Thr Asp Ile Gln Gly Glu Glu
485                 490                 495                 500

ttt gcc aaa gtt ata gta caa aaa ttg aaa gat aac aaa caa atg ggt 1772
Phe Ala Lys Val Ile Val Gln Lys Leu Lys Asp Asn Lys Gln Met Gly
                505                 510                 515

ctt cag cct tac ccg gaa ata ctt gtg gtt tct aga tca cca tct tta 1820
Leu Gln Pro Tyr Pro Glu Ile Leu Val Val Ser Arg Ser Pro Ser Leu
            520                 525                 530

aat tta ctt caa aat aaa agc atg taa gtgactgttt ttcaagaaga 1867
Asn Leu Leu Gln Asn Lys Ser Met
        535                 540

aatgtgtttc ataaaaggat atttatatct ctgttgcttt gacttttttt atataaaatc 1927

cgtgagtatt aaagctttat tgaaggttct ttgggtaaat attagtctcc ctccatgaca 1987

ctgcagtatt ttttttaatt aatacaagta aaaagttgaa tttggttgaa tttgctacat 2047

agttcaattt ttatgtctct tttgttaaca gaaaccactt ttaaaggata gtaattattc 2107

ttgtttataa cagtgcctta aggtatgatg tatttctgat ggaagccatt ttcacattca 2167

tgttcttcat ggattatttg ttacttgtct aagatgcaat ttgattttat gaagtatata 2227

ccctttaccc accagagaca gtacagaatc cctgccctaa aatcccaggc ttaattgccc 2287

tacaaagggt tattaattta aaactccatt attaggatta cattttaaag ttttatttat 2347

gaattccctt taaaaatgat atttcaaagg taaaacaata caatataaag aaaaaaataa 2407

atatattaat accggcttcc tgtccccatt tttaacctca gccttcccta ctgtcaccaa 2467

caaccaagct aaataaagtc aacagcctga tgtg                                    2501

<210> 2
<211> 540
<212> PRT
<213> Homo sapiens

<400> 2
Met Asn Gly Glu Ala Ile Cys Ser Ala Leu Pro Thr Ile Pro Tyr His
1               5                   10                  15
Lys Leu Ala Asp Leu Arg Tyr Leu Ser Arg Gly Ala Ser Gly Thr Val
            20                  25                  30
Ser Ser Ala Arg His Ala Asp Trp Arg Val Gln Val Ala Val Lys His
        35                  40                  45
Leu His Ile His Thr Pro Leu Leu Asp Ser Glu Arg Lys Asp Val Leu
    50                  55                  60
Arg Glu Ala Glu Ile Leu His Lys Ala Arg Phe Ser Tyr Ile Leu Pro
65                  70                  75                  80
Ile Leu Gly Ile Cys Asn Glu Pro Glu Phe Leu Gly Ile Val Thr Glu
                85                  90                  95
Tyr Met Pro Asn Gly Ser Leu Asn Glu Leu Leu His Arg Lys Thr Glu
            100                 105                 110
Tyr Pro Asp Val Ala Trp Pro Leu Arg Phe Arg Ile Leu His Glu Ile
        115                 120                 125
Ala Leu Gly Val Asn Tyr Leu His Asn Met Thr Pro Pro Leu Leu His
    130                 135                 140
His Asp Leu Lys Thr Gln Asn Ile Leu Leu Asp Asn Glu Phe His Val
145                 150                 155                 160
Lys Ile Ala Asp Phe Gly Leu Ser Lys Trp Arg Met Met Ser Leu Ser
                165                 170                 175
Gln Ser Arg Ser Ser Lys Ser Ala Pro Glu Gly Gly Thr Ile Ile Tyr
            180                 185                 190
Met Pro Pro Glu Asn Tyr Glu Pro Gly Gln Lys Ser Arg Ala Ser Ile
        195                 200                 205
Lys His Asp Ile Tyr Ser Tyr Ala Val Ile Thr Trp Glu Val Leu Ser
    210                 215                 220
Arg Lys Gln Pro Phe Glu Asp Val Thr Asn Pro Leu Gln Ile Met Tyr
225                 230                 235                 240
Ser Val Ser Gln Gly His Arg Pro Val Ile Asn Glu Glu Ser Leu Pro
                245                 250                 255
Tyr Asp Ile Pro His Arg Ala Arg Met Ile Ser Leu Ile Glu Ser Gly
            260                 265                 270
Trp Ala Gln Asn Pro Asp Glu Arg Pro Ser Phe Leu Lys Cys Leu Ile
        275                 280                 285
Glu Leu Glu Pro Val Leu Arg Thr Phe Glu Glu Ile Thr Phe Leu Glu
    290                 295                 300
Ala Val Ile Gln Leu Lys Lys Thr Lys Leu Gln Ser Val Ser Ser Ala
305                 310                 315                 320
Ile His Leu Cys Asp Lys Lys Lys Met Glu Leu Ser Leu Asn Ile Pro
                325                 330                 335
Val Asn His Gly Pro Gln Glu Glu Ser Cys Gly Ser Ser Gln Leu His
            340                 345                 350
Glu Asn Ser Gly Ser Pro Glu Thr Ser Arg Ser Leu Pro Ala Pro Gln
        355                 360                 365
Asp Asn Asp Phe Leu Ser Arg Lys Ala Gln Asp Cys Tyr Phe Met Lys
    370                 375                 380
Leu His His Cys Pro Gly Asn His Ser Trp Asp Ser Thr Ile Ser Gly
385                 390                 395                 400
Ser Gln Arg Ala Ala Phe Cys Asp His Lys Thr Thr Pro Cys Ser Ser
                405                 410                 415
Ala Ile Ile Asn Pro Leu Ser Thr Ala Gly Asn Ser Glu Arg Leu Gln
            420                 425                 430
Pro Gly Ile Ala Gln Gln Trp Ile Gln Ser Lys Arg Glu Asp Ile Val

        435                 440                 445
Asn Gln Met Thr Glu Ala Cys Leu Asn Gln Ser Leu Asp Ala Leu Leu
    450                 455                 460
Ser Arg Asp Leu Ile Met Lys Glu Asp Tyr Glu Leu Val Ser Thr Lys
465                 470                 475                 480
Pro Thr Arg Thr Ser Lys Val Arg Gln Leu Leu Asp Thr Thr Asp Ile
                485                 490                 495
Gln Gly Glu Glu Phe Ala Lys Val Ile Val Gln Lys Leu Lys Asp Asn
            500                 505                 510
Lys Gln Met Gly Leu Gln Pro Tyr Pro Glu Ile Leu Val Val Ser Arg
        515                 520                 525
Ser Pro Ser Leu Asn Leu Leu Gln Asn Lys Ser Met
    530                 535                 540

SEQUENCE LISTING

<110> Axxima Pharmaceuticals AG

<120> CELLULAR KINASES INVOLVED IN CYTOMEGALOVIRUS INFECTION
      AND THEIR INHIBITION

<130> U50062

<140>
<141>

<160> 2

<170> PatentIn Ver. 2.1

<210> 1
<211> 2617
<212> DNA
<213> Homo sapiens

<220>
<221> CDS
<222> (1)..(2016)

<400> 1
atg caa cca gac atg tcc ttg aat gtc att aag atg aaa tcc agt gac    48
Met Gln Pro Asp Met Ser Leu Asn Val Ile Lys Met Lys Ser Ser Asp
  1               5                  10                  15

ttc ctg gag agt gca gaa ctg gac agc gga ggc ttt ggg aag gtg tct    96
Phe Leu Glu Ser Ala Glu Leu Asp Ser Gly Gly Phe Gly Lys Val Ser
             20                  25                  30

ctg tgt ttc cac aga acc cag gga ctc atg atc atg aaa aca gtg tac    144
Leu Cys Phe His Arg Thr Gln Gly Leu Met Ile Met Lys Thr Val Tyr
         35                  40                  45

aag ggg ccc aac tgc att gag cac aac gag gcc ctc ttg gag gag gcg    192
Lys Gly Pro Asn Cys Ile Glu His Asn Glu Ala Leu Leu Glu Glu Ala
     50                  55                  60

aag atg atg aac aga ctg aga cac agc cgg gtg gtg aag ctc ctg ggc    240
Lys Met Met Asn Arg Leu Arg His Ser Arg Val Val Lys Leu Leu Gly
 65                  70                  75                  80

gtc atc ata gag gaa ggg aag tac tcc ctg gtg atg gag tac atg gag    288
Val Ile Ile Glu Glu Gly Lys Tyr Ser Leu Val Met Glu Tyr Met Glu
                 85                  90                  95

aag ggc aac ctg atg cac gtg ctg aaa gcc gag atg agt act ccg ctt     336
Lys Gly Asn Leu Met His Val Leu Lys Ala Glu Met Ser Thr Pro Leu
            100                 105                 110

tct gta aaa gga agg ata att ttg gaa atc att gaa gga atg tgc tac     384
Ser Val Lys Gly Arg Ile Ile Leu Glu Ile Ile Glu Gly Met Cys Tyr
        115                 120                 125

tta cat gga aaa ggc gtg ata cac aag gac ctg aag cct gaa aat atc     432
Leu His Gly Lys Gly Val Ile His Lys Asp Leu Lys Pro Glu Asn Ile
    130                 135                 140

ctt gtt gat aat gac ttc cac att aag atc gca gac ctc ggc ctt gcc     480
Leu Val Asp Asn Asp Phe His Ile Lys Ile Ala Asp Leu Gly Leu Ala
145                 150                 155                 160

tcc ttt aag atg tgg agc aaa ctg aat aat gaa gag cac aat gag ctg     528
Ser Phe Lys Met Trp Ser Lys Leu Asn Asn Glu Glu His Asn Glu Leu
                165                 170                 175

agg gaa gtg gac ggc acc gct aag aag aat ggc ggc acc ctc tac tac     576
Arg Glu Val Asp Gly Thr Ala Lys Lys Asn Gly Gly Thr Leu Tyr Tyr
            180                 185                 190

atg gcg ccc gag cac ctg aat gac gtc aac gca aag ccc aca gag aag     624
Met Ala Pro Glu His Leu Asn Asp Val Asn Ala Lys Pro Thr Glu Lys
        195                 200                 205

tcg gat gtg tac agc ttt gct gta gta ctc tgg gcg ata ttt gca aat     672
Ser Asp Val Tyr Ser Phe Ala Val Val Leu Trp Ala Ile Phe Ala Asn
    210                 215                 220

aag gag cca tat gaa aat gct atc tgt gag cag cag ttg ata atg tgc     720
Lys Glu Pro Tyr Glu Asn Ala Ile Cys Glu Gln Gln Leu Ile Met Cys
225                 230                 235                 240

ata aaa tct ggg aac agg cca gat gtg gat gac atc act gag tac tgc     768
Ile Lys Ser Gly Asn Arg Pro Asp Val Asp Asp Ile Thr Glu Tyr Cys
                245                 250                 255

cca aga gaa att atc agt ctc atg aag ctc tgc tgg gaa gcg aat ccg     816
Pro Arg Glu Ile Ile Ser Leu Met Lys Leu Cys Trp Glu Ala Asn Pro
            260                 265                 270

gaa gct cgg ccg aca ttt cct ggc att gaa gaa aaa ttt agg cct ttt     864
Glu Ala Arg Pro Thr Phe Pro Gly Ile Glu Glu Lys Phe Arg Pro Phe
        275                 280                 285

tat tta agt caa tta gaa gaa agt gta gaa gag gac gtg aag agt tta     912
Tyr Leu Ser Gln Leu Glu Glu Ser Val Glu Glu Asp Val Lys Ser Leu
    290                 295                 300

aag aaa gag tat tca aac gaa aat gca gtt gtg aag aga atg cag tct     960
Lys Lys Glu Tyr Ser Asn Glu Asn Ala Val Val Lys Arg Met Gln Ser
305                 310                 315                 320

ctt caa ctt gat tgt gtg gca gta cct tca agc cgg tca aat tca gcc     1008
Leu Gln Leu Asp Cys Val Ala Val Pro Ser Ser Arg Ser Asn Ser Ala
                325                 330                 335

aca gaa cag cct ggt tca ctg cac agt tcc cag gga ctt ggg atg ggt     1056
Thr Glu Gln Pro Gly Ser Leu His Ser Ser Gln Gly Leu Gly Met Gly
            340                 345                 350

cct gtg gag gag tcc tgg ttt gct cct tcc ctg gag cac cca caa gaa     1104
Pro Val Glu Glu Ser Trp Phe Ala Pro Ser Leu Glu His Pro Gln Glu
        355                 360                 365

gag aat gag ccc agc ctg cag agt aaa ctc caa gac gaa gcc aac tac     1152
Glu Asn Glu Pro Ser Leu Gln Ser Lys Leu Gln Asp Glu Ala Asn Tyr
    370                 375                 380

cat ctt tat ggc agc cgc atg gac agg cag acg aaa cag cag ccc aga     1200
His Leu Tyr Gly Ser Arg Met Asp Arg Gln Thr Lys Gln Gln Pro Arg
385                 390                 395                 400

cag aat gtg gct tac aac aga gag gag gaa agg aga cgc agg gtc tcc     1248
Gln Asn Val Ala Tyr Asn Arg Glu Glu Glu Arg Arg Arg Arg Val Ser
                405                 410                 415

cat gac cct ttt gca cag caa aga cct tac gag aat ttt cag aat aca     1296
His Asp Pro Phe Ala Gln Gln Arg Pro Tyr Glu Asn Phe Gln Asn Thr
            420                 425                 430

gag gga aaa ggc act gtt tat tcc agt gca gcc agt cat ggt aat gca     1344
Glu Gly Lys Gly Thr Val Tyr Ser Ser Ala Ala Ser His Gly Asn Ala
        435                 440                 445

gtg cac cag ccc tca ggg ctc acc agc caa cct caa gta ctg tat cag     1392
Val His Gln Pro Ser Gly Leu Thr Ser Gln Pro Gln Val Leu Tyr Gln
    450                 455                 460

aac aat gga tta tat agc tca cat ggc ttt gga aca aga cca ctg gat     1440
Asn Asn Gly Leu Tyr Ser Ser His Gly Phe Gly Thr Arg Pro Leu Asp
465                 470                 475                 480

cca gga aca gca ggt ccc aga gtt tgg tac agg cca att cca agt cat     1488
Pro Gly Thr Ala Gly Pro Arg Val Trp Tyr Arg Pro Ile Pro Ser His
                485                 490                 495

atg cct agt ctg cat aat atc cca gtg cct gag acc aac tat cta gga     1536
Met Pro Ser Leu His Asn Ile Pro Val Pro Glu Thr Asn Tyr Leu Gly
            500                 505                 510

aat aca ccc acc atg cca ttc agc tcc ttg cca cca aca gat gaa tct     1584
Asn Thr Pro Thr Met Pro Phe Ser Ser Leu Pro Pro Thr Asp Glu Ser
        515                 520                 525

ata aaa tat acc ata tac aat agt act ggc att cag att gga gcc tac     1632
Ile Lys Tyr Thr Ile Tyr Asn Ser Thr Gly Ile Gln Ile Gly Ala Tyr
    530                 535                 540

aat tat atg gag att ggt ggg acg agt tca tca cta cta gac agc aca     1680
Asn Tyr Met Glu Ile Gly Gly Thr Ser Ser Ser Leu Leu Asp Ser Thr
545                 550                 555                 560

aat acg aac ttc aaa gaa gag cca gct gct aag tac caa gct atc ttt     1728
Asn Thr Asn Phe Lys Glu Glu Pro Ala Ala Lys Tyr Gln Ala Ile Phe
                565                 570                 575

gat aat acc act agt ctg acg gat aaa cac ctg gac cca atc agg gaa     1776
Asp Asn Thr Thr Ser Leu Thr Asp Lys His Leu Asp Pro Ile Arg Glu
            580                 585                 590

aat ctg gga aag cac tgg aaa aac tgt gcc cgt aaa ctg ggc ttc aca     1824
Asn Leu Gly Lys His Trp Lys Asn Cys Ala Arg Lys Leu Gly Phe Thr
        595                 600                 605

cag tct cag att gat gaa att gac cat gac tat gag cga gat gga ctg 1872
Gln Ser Gln Ile Asp Glu Ile Asp His Asp Tyr Glu Arg Asp Gly Leu
    610                 615                 620

aaa gaa aag gtt tac cag atg ctc caa aag tgg gtg atg agg gaa ggc 1920
Lys Glu Lys Val Tyr Gln Met Leu Gln Lys Trp Val Met Arg Glu Gly
625                 630                 635                 640

ata aag gga gcc acg gtg ggg aag ctg gcc cag gcg ctc cac cag tgt 1968
Ile Lys Gly Ala Thr Val Gly Lys Leu Ala Gln Ala Leu His Gln Cys
                645                 650                 655

tcc agg atc gac ctt ctg agc agc ttg att tac gtc agc cag aac taa 2016
Ser Arg Ile Asp Leu Leu Ser Ser Leu Ile Tyr Val Ser Gln Asn
            660                 665                 670

ccctggatgg gctacggcag ctgaagtgga cgcctcactt agcggataac cccagaaagt 2076

tggctgcctc agagcattca gaattctgtc ctcactgata ggggttctgt gtctgcagaa 2136

atttngtttc ctgtacttca tagctggaga atggggaaag aaatctgcag caaaggggtc 2196

tcactctgtt gccaggctgg tctcaaactt ctggactcaa gtgatcctcc cgcctcggcc 2256

ttccaaagtg ctgggatatc aggcactgag ccactgcgcc cagtcaacaa tccgntctga 2316

ggaaagcgta agcaggaaga cctcttaatg gcatagcacc aataaaaaaa tgactcctag 2376

ttgtgtttgg aaagggagag aagagatgtc tgaggaaggt catgttcttt cagcttatgg 2436

catttcctag agtttngttg aagcaagaag aaaaactcag agaatataaa atcaactttn 2496

aaaattgtgt gctctcttct tcacgtaggc tcctgttaaa aacaaagtgc agtcagattc 2556

taagccctgt tcagagactt cgcggatcac agctgcagct caccgccaca tcacaggatc 2616

c 2617

<210> 2
<211> 671
<212> PRT
<213> Homo sapiens

<400> 2
Met Gln Pro Asp Met Ser Leu Asn Val Ile Lys Met Lys Ser Ser Asp
1               5                   10                  15
Phe Leu Glu Ser Ala Glu Leu Asp Ser Gly Gly Phe Gly Lys Val Ser
            20                  25                  30
Leu Cys Phe His Arg Thr Gln Gly Leu Met Ile Met Lys Thr Val Tyr
        35                  40                  45
Lys Gly Pro Asn Cys Ile Glu His Asn Glu Ala Leu Leu Glu Glu Ala
    50                  55                  60
Lys Met Met Asn Arg Leu Arg His Ser Arg Val Val Lys Leu Leu Gly
65                  70                  75                  80
Val Ile Ile Glu Glu Gly Lys Tyr Ser Leu Val Met Glu Tyr Met Glu
                85                  90                  95
Lys Gly Asn Leu Met His Val Leu Lys Ala Glu Met Ser Thr Pro Leu
            100                 105                 110
Ser Val Lys Gly Arg Ile Ile Leu Glu Ile Ile Glu Gly Met Cys Tyr
        115                 120                 125
Leu His Gly Lys Gly Val Ile His Lys Asp Leu Lys Pro Glu Asn Ile
    130                 135                 140
Leu Val Asp Asn Asp Phe His Ile Lys Ile Ala Asp Leu Gly Leu Ala
145                 150                 155                 160

Ser Phe Lys Met Trp Ser Lys Leu Asn Asn Glu Glu His Asn Glu Leu
                165                 170                 175
Arg Glu Val Asp Gly Thr Ala Lys Lys Asn Gly Gly Thr Leu Tyr Tyr
            180                 185                 190
Met Ala Pro Glu His Leu Asn Asp Val Asn Ala Lys Pro Thr Glu Lys
        195                 200                 205
Ser Asp Val Tyr Ser Phe Ala Val Val Leu Trp Ala Ile Phe Ala Asn
    210                 215                 220
Lys Glu Pro Tyr Glu Asn Ala Ile Cys Glu Gln Gln Leu Ile Met Cys
225                 230                 235                 240
Ile Lys Ser Gly Asn Arg Pro Asp Val Asp Asp Ile Thr Glu Tyr Cys
                245                 250                 255
Pro Arg Glu Ile Ile Ser Leu Met Lys Leu Cys Trp Glu Ala Asn Pro
            260                 265                 270
Glu Ala Arg Pro Thr Phe Pro Gly Ile Glu Glu Lys Phe Arg Pro Phe
        275                 280                 285
Tyr Leu Ser Gln Leu Glu Glu Ser Val Glu Glu Asp Val Lys Ser Leu
    290                 295                 300
Lys Lys Glu Tyr Ser Asn Glu Asn Ala Val Val Lys Arg Met Gln Ser
305                 310                 315                 320
Leu Gln Leu Asp Cys Val Ala Val Pro Ser Ser Arg Ser Asn Ser Ala
                325                 330                 335
Thr Glu Gln Pro Gly Ser Leu His Ser Ser Gln Gly Leu Gly Met Gly
            340                 345                 350
Pro Val Glu Glu Ser Trp Phe Ala Pro Ser Leu Glu His Pro Gln Glu
        355                 360                 365
Glu Asn Glu Pro Ser Leu Gln Ser Lys Leu Gln Asp Glu Ala Asn Tyr
    370                 375                 380
His Leu Tyr Gly Ser Arg Met Asp Arg Gln Thr Lys Gln Gln Pro Arg
385                 390                 395                 400
Gln Asn Val Ala Tyr Asn Arg Glu Glu Glu Arg Arg Arg Arg Val Ser
                405                 410                 415
His Asp Pro Phe Ala Gln Gln Arg Pro Tyr Glu Asn Phe Gln Asn Thr
            420                 425                 430
Glu Gly Lys Gly Thr Val Tyr Ser Ser Ala Ala Ser His Gly Asn Ala
        435                 440                 445
Val His Gln Pro Ser Gly Leu Thr Ser Gln Pro Gln Val Leu Tyr Gln
    450                 455                 460
Asn Asn Gly Leu Tyr Ser Ser His Gly Phe Gly Thr Arg Pro Leu Asp
465                 470                 475                 480
Pro Gly Thr Ala Gly Pro Arg Val Trp Tyr Arg Pro Ile Pro Ser His
                485                 490                 495
Met Pro Ser Leu His Asn Ile Pro Val Pro Glu Thr Asn Tyr Leu Gly
            500                 505                 510
Asn Thr Pro Thr Met Pro Phe Ser Ser Leu Pro Pro Thr Asp Glu Ser
        515                 520                 525
Ile Lys Tyr Thr Ile Tyr Asn Ser Thr Gly Ile Gln Ile Gly Ala Tyr
    530                 535                 540
Asn Tyr Met Glu Ile Gly Gly Thr Ser Ser Ser Leu Leu Asp Ser Thr
545                 550                 555                 560
Asn Thr Asn Phe Lys Glu Glu Pro Ala Ala Lys Tyr Gln Ala Ile Phe
                565                 570                 575
Asp Asn Thr Thr Ser Leu Thr Asp Lys His Leu Asp Pro Ile Arg Glu
            580                 585                 590
Asn Leu Gly Lys His Trp Lys Asn Cys Ala Arg Lys Leu Gly Phe Thr
        595                 600                 605
Gln Ser Gln Ile Asp Glu Ile Asp His Asp Tyr Glu Arg Asp Gly Leu
    610                 615                 620
Lys Glu Lys Val Tyr Gln Met Leu Gln Lys Trp Val Met Arg Glu Gly
625                 630                 635                 640
Ile Lys Gly Ala Thr Val Gly Lys Leu Ala Gln Ala Leu His Gln Cys
                645                 650                 655
Ser Arg Ile Asp Leu Leu Ser Ser Leu Ile Tyr Val Ser Gln Asn
            660                 665                 670

SEQUENCE LISTING

<110> Axxima Pharmaceuticals AG

<120> CELLULAR KINASES INVOLVED IN CYTOMEGALOVIRUS INFECTION
      AND THEIR INHIBITION

<130> Y10256

<140>
<141>

<160> 2

<170> PatentIn Ver. 2.1

<210> 1
<211> 4596
<212> DNA
<213> Homo sapiens

<220>
<221> CDS
<222> (233)..(3076)

<400> 1
aagcggggga ctgtgccgtg tggaacgtgt agctgttgag aggtggactc tgttaccatt 60

gaggatgttt ggaggatgag tatgtgtggc agaggcacac ataaacaggc agagaccctt 120

tgcccctgcc tttctccccc aacccaaggc tgacctgtgt tctcccaggt ctgggattct 180

aagtgacctg ctctgtgttt ggtctctctc aggatgagca caagcctggg ag atg gca 238
                                                          Met Ala
                                                            1

gtg atg gaa atg gcc tgc cca ggt gcc cct ggc tca gca gtg ggg cag    286
Val Met Glu Met Ala Cys Pro Gly Ala Pro Gly Ser Ala Val Gly Gln
          5                  10                  15

cag aag gaa ctc ccc aag cca aag gag aag acg ccg cca ctg ggg aag    334
Gln Lys Glu Leu Pro Lys Pro Lys Glu Lys Thr Pro Pro Leu Gly Lys
     20                  25                  30

aaa cag agc tcc gtc tac aag ctt gag gcc gtg gag aag agc cct gtg    382
Lys Gln Ser Ser Val Tyr Lys Leu Glu Ala Val Glu Lys Ser Pro Val
 35                  40                  45                  50

ttc tgc gga aag tgg gag atc ctg aat gac gtg att acc aag ggc aca    430
Phe Cys Gly Lys Trp Glu Ile Leu Asn Asp Val Ile Thr Lys Gly Thr
                 55                  60                  65

gcc aag gaa ggc tcc gag gca ggg cca gct gcc atc tct atc atc gcc    478
Ala Lys Glu Gly Ser Glu Ala Gly Pro Ala Ala Ile Ser Ile Ile Ala
             70                  75                  80

cag gct gag tgt gag aat agc caa gag ttc agc ccc acc ttt tca gaa    526
Gln Ala Glu Cys Glu Asn Ser Gln Glu Phe Ser Pro Thr Phe Ser Glu
         85                  90                  95

cgc att ttc atc gct ggg tcc aaa cag tac agc cag tcc gag agt ctt    574
Arg Ile Phe Ile Ala Gly Ser Lys Gln Tyr Ser Gln Ser Glu Ser Leu
    100                 105                 110

gat cag atc ccc aac aat gtg gcc cat gct aca gag ggc aaa atg gcc 622
Asp Gln Ile Pro Asn Asn Val Ala His Ala Thr Glu Gly Lys Met Ala
115                 120                 125                 130

cgt gtg tgt tgg aag gga aag cgt cgc agc aaa gcc cgg aag aaa cgg 670
Arg Val Cys Trp Lys Gly Lys Arg Arg Ser Lys Ala Arg Lys Lys Arg
                135                 140                 145

aag aag aag agc tca aag tcc ctg gct cat gca gga gtg gcc ttg gcc 718
Lys Lys Lys Ser Ser Lys Ser Leu Ala His Ala Gly Val Ala Leu Ala
            150                 155                 160

aaa ccc ctc ccc agg acc cct gag cag gag agc tgc acc atc cca gtg 766
Lys Pro Leu Pro Arg Thr Pro Glu Gln Glu Ser Cys Thr Ile Pro Val
        165                 170                 175

cag gag gat gag tct cca ctc ggc gcc cca tat gtt aga aac acc ccg 814
Gln Glu Asp Glu Ser Pro Leu Gly Ala Pro Tyr Val Arg Asn Thr Pro
    180                 185                 190

cag ttc acc aag cct ctg aag gaa cca ggc ctt ggg caa ctc tgt ttt 862
Gln Phe Thr Lys Pro Leu Lys Glu Pro Gly Leu Gly Gln Leu Cys Phe
195                 200                 205                 210

aag cag ctt ggc gag ggc cta cgg ccg gct ctg cct cga tca gaa ctc 910
Lys Gln Leu Gly Glu Gly Leu Arg Pro Ala Leu Pro Arg Ser Glu Leu
                215                 220                 225

cac aaa ctg atc agc ccc ttg caa tgt ctg aac cac gtg tgg aaa ctg 958
His Lys Leu Ile Ser Pro Leu Gln Cys Leu Asn His Val Trp Lys Leu
            230                 235                 240

cac cac ccc cag gac gga ggc ccc ctg ccc ctg ccc acg cac ccc ttc 1006
His His Pro Gln Asp Gly Gly Pro Leu Pro Leu Pro Thr His Pro Phe
        245                 250                 255

ccc tat agc aga ctg cct cat ccc ttc cca ttc cac cct ctc cag ccc 1054
Pro Tyr Ser Arg Leu Pro His Pro Phe Pro Phe His Pro Leu Gln Pro
    260                 265                 270

tgg aaa cct cac cct ctg gag tcc ttc ctg ggc aaa ctg gcc tgt gta 1102
Trp Lys Pro His Pro Leu Glu Ser Phe Leu Gly Lys Leu Ala Cys Val
275                 280                 285                 290

gac agc cag aaa ccc ttg cct gac cca cac ctg agc aaa ctg gcc tgt 1150
Asp Ser Gln Lys Pro Leu Pro Asp Pro His Leu Ser Lys Leu Ala Cys
                295                 300                 305

gta gac agt cca aag ccc ctg cct ggc cca cac ctg gag ccc agc tgc 1198
Val Asp Ser Pro Lys Pro Leu Pro Gly Pro His Leu Glu Pro Ser Cys
            310                 315                 320

ctg tct cgt ggt gcc cat gag aag ttt tct gtg gag gaa tac cta gtg 1246
Leu Ser Arg Gly Ala His Glu Lys Phe Ser Val Glu Glu Tyr Leu Val
        325                 330                 335

cat gct ctg caa ggc agc gtg agc tca agc cag gcc cac agc ctg acc 1294
His Ala Leu Gln Gly Ser Val Ser Ser Ser Gln Ala His Ser Leu Thr
    340                 345                 350

agc ctg gcc aag acc tgg gca gca cgg ggc tcc aga tcc cgg gag ccc 1342
Ser Leu Ala Lys Thr Trp Ala Ala Arg Gly Ser Arg Ser Arg Glu Pro
355                 360                 365                 370

agc ccc aaa act gag gac aac gag ggt gtc ctg ctc act gag aaa ctc 1390
Ser Pro Lys Thr Glu Asp Asn Glu Gly Val Leu Leu Thr Glu Lys Leu
                375                 380                 385

aag cca gtg gat tat gag tac cga gaa gaa gtc cac tgg gcc acg cac 1438
Lys Pro Val Asp Tyr Glu Tyr Arg Glu Glu Val His Trp Ala Thr His
            390                 395                 400

cag ctc cgc ctg ggc aga ggc tcc ttc gga gag gtg cac agg atg gag 1486
Gln Leu Arg Leu Gly Arg Gly Ser Phe Gly Glu Val His Arg Met Glu
        405                 410                 415

gac aag cag act ggc ttc cag tgc gct gtc aaa aag gtg cgg ctg gaa 1534
Asp Lys Gln Thr Gly Phe Gln Cys Ala Val Lys Lys Val Arg Leu Glu
    420                 425                 430

gta ttt cgg gca gag gag ctg atg gca tgt gca gga ttg acc tca ccc 1582
Val Phe Arg Ala Glu Glu Leu Met Ala Cys Ala Gly Leu Thr Ser Pro
435                 440                 445                 450

aga att gtc cct ttg tat gga gct gtg aga gaa ggg cct tgg gtc aac 1630
Arg Ile Val Pro Leu Tyr Gly Ala Val Arg Glu Gly Pro Trp Val Asn
                455                 460                 465

atc ttc atg gag ctg ctg gaa ggt ggc tcc ctg ggc cag ctg gtc aag 1678
Ile Phe Met Glu Leu Leu Glu Gly Gly Ser Leu Gly Gln Leu Val Lys
            470                 475                 480

gag cag ggc tgt ctc cca gag gac cgg gcc ctg tac tac ctg ggc cag 1726
Glu Gln Gly Cys Leu Pro Glu Asp Arg Ala Leu Tyr Tyr Leu Gly Gln
        485                 490                 495

gcc ctg gag ggt ctg gaa tac ctc cac tca cga agg att ctg cat ggg 1774
Ala Leu Glu Gly Leu Glu Tyr Leu His Ser Arg Arg Ile Leu His Gly
    500                 505                 510

gac gtc aaa gct gac aac gtg ctc ctg tcc agc gat ggg agc cac gca 1822
Asp Val Lys Ala Asp Asn Val Leu Leu Ser Ser Asp Gly Ser His Ala
515                 520                 525                 530

gcc ctc tgt gac ttt ggc cat gct gtg tgt ctt caa cct gat ggc ctg 1870
Ala Leu Cys Asp Phe Gly His Ala Val Cys Leu Gln Pro Asp Gly Leu
                535                 540                 545

gga aag tcc ttg ctc aca ggg gac tac atc cct ggc aca gag acc cac 1918
Gly Lys Ser Leu Leu Thr Gly Asp Tyr Ile Pro Gly Thr Glu Thr His
            550                 555                 560

atg gct ccg gag gtg gtg ctg ggc agg agc tgc gac gcc aag gtg gat 1966
Met Ala Pro Glu Val Val Leu Gly Arg Ser Cys Asp Ala Lys Val Asp
        565                 570                 575

gtc tgg agc agc tgc tgt atg atg ctg cac atg ctc aac ggc tgc cac 2014
Val Trp Ser Ser Cys Cys Met Met Leu His Met Leu Asn Gly Cys His
    580                 585                 590

ccc tgg act cag ttc ttc cga ggg ccg ctc tgc ctc aag att gcc agc 2062
Pro Trp Thr Gln Phe Phe Arg Gly Pro Leu Cys Leu Lys Ile Ala Ser
595                 600                 605                 610

gag cct ccg cct gtg agg gag atc cca ccc tcc tgc gcc cct ctc aca 2110
Glu Pro Pro Pro Val Arg Glu Ile Pro Pro Ser Cys Ala Pro Leu Thr
                615                 620                 625

gcc cag gcc atc caa gag ggg ctg agg aaa gag ccc atc cac cgc gtg    2158
Ala Gln Ala Ile Gln Glu Gly Leu Arg Lys Glu Pro Ile His Arg Val
            630                 635                 640

tct gca gcg gag ctg gga ggg aag gtg aac cgg gca cta cag caa gtg    2206
Ser Ala Ala Glu Leu Gly Gly Lys Val Asn Arg Ala Leu Gln Gln Val
        645                 650                 655

gga ggt ctg aag agc cct tgg agg gga gaa tat aaa gaa cca aga cat    2254
Gly Gly Leu Lys Ser Pro Trp Arg Gly Glu Tyr Lys Glu Pro Arg His
    660                 665                 670

cca ccg cca aat caa gcc aat tac cac cag acc ctc cat gcc cag ccg    2302
Pro Pro Pro Asn Gln Ala Asn Tyr His Gln Thr Leu His Ala Gln Pro
675                 680                 685                 690

aga gag ctt tcg cca agg gcc cca ggg ccc cgg cca gct gag gag aca    2350
Arg Glu Leu Ser Pro Arg Ala Pro Gly Pro Arg Pro Ala Glu Glu Thr
                695                 700                 705

aca ggc aga gcc cct aag ctc cag cct cct ctc cca cca gag ccc cca    2398
Thr Gly Arg Ala Pro Lys Leu Gln Pro Pro Leu Pro Pro Glu Pro Pro
            710                 715                 720

gag cca aac aag tct cct ccc ttg act ttg agc aag gag gag tct ggg    2446
Glu Pro Asn Lys Ser Pro Pro Leu Thr Leu Ser Lys Glu Glu Ser Gly
        725                 730                 735

atg tgg gaa ccc tta cct ctg tcc tcc ctg gag cca gcc cct gcc aga    2494
Met Trp Glu Pro Leu Pro Leu Ser Ser Leu Glu Pro Ala Pro Ala Arg
    740                 745                 750

aac ccc agc tca cca gag cgg aaa gca acc gtc ccg gag cag gaa ctg    2542
Asn Pro Ser Ser Pro Glu Arg Lys Ala Thr Val Pro Glu Gln Glu Leu
755                 760                 765                 770

cag cag ctg gaa ata gaa tta ttc ctc aac agc ctg tcc cag cca ttt    2590
Gln Gln Leu Glu Ile Glu Leu Phe Leu Asn Ser Leu Ser Gln Pro Phe
                775                 780                 785

tct ctg gag gag cag gag caa att ctc tcg tgc ctc agc atc gac agc    2638
Ser Leu Glu Glu Gln Glu Gln Ile Leu Ser Cys Leu Ser Ile Asp Ser
            790                 795                 800

ctc tcc ctg tcg gat gac agt gag aag aac cca tca aag gcc tct caa    2686
Leu Ser Leu Ser Asp Asp Ser Glu Lys Asn Pro Ser Lys Ala Ser Gln
        805                 810                 815

agc tcg cgg gac acc ctg agc tca ggc gta cac tcc tgg agc agc cag    2734
Ser Ser Arg Asp Thr Leu Ser Ser Gly Val His Ser Trp Ser Ser Gln
    820                 825                 830

gcc gag gct cga agc tcc agc tgg aac atg gtg ctg gcc cgg ggg cgg    2782
Ala Glu Ala Arg Ser Ser Ser Trp Asn Met Val Leu Ala Arg Gly Arg
835                 840                 845                 850

ccc acc gac acc cca agc tat ttc aat ggt gtg aaa gtc caa ata cag    2830
Pro Thr Asp Thr Pro Ser Tyr Phe Asn Gly Val Lys Val Gln Ile Gln
                855                 860                 865

tct ctt aat ggt gaa cac ctg cac atc cgg gag ttc cac cgg gtc aaa    2878
Ser Leu Asn Gly Glu His Leu His Ile Arg Glu Phe His Arg Val Lys
            870                 875                 880

gtg gga gac atc gcc act ggc atc agc agc cag atc cca gct gca gcc    2926
Val Gly Asp Ile Ala Thr Gly Ile Ser Ser Gln Ile Pro Ala Ala Ala
        885                 890                 895

ttc agc ttg gtc acc aaa gac ggg cag cct gtt cgc tac gac atg gag    2974
Phe Ser Leu Val Thr Lys Asp Gly Gln Pro Val Arg Tyr Asp Met Glu
    900                 905                 910

gtg cca gac tcg ggc atc gac ctg cag tgc aca ctg gcc cct gat ggc    3022
Val Pro Asp Ser Gly Ile Asp Leu Gln Cys Thr Leu Ala Pro Asp Gly
915                 920                 925                 930

agc ttc gcc tgg agc tgg agg gtc aag cat ggc cag ctg gag aac agg    3070
Ser Phe Ala Trp Ser Trp Arg Val Lys His Gly Gln Leu Glu Asn Arg
                935                 940                 945

ccc taa ccctgccctc caccgccggc tccacactgc cggaaagcag ccttcctgct    3126
Pro

cggtgcacga tgctgccctg aaaacacagg ctcagccgtt cccaggggat tgccagcccc 3186

ccggctcaca gtgggaacca gggcctcgca gcagcaaggt gggggcaagc agaatgcctc 3246

ccaggatttc acacctgagc cctgccccac cctgctgaaa aaacatccgc cacgtgaaga 3306

gacagaagga ggatggcagg agttacctgg ggaaacaaaa cagggatctt tttctgcccc 3366

tgctccagtc gagttggcct gacccgcttg gatcagtgac catttgttgg cagacagggg 3426

agagcagctt ccagcctggg tcagaagggg tgggcgagcc cttcggcccc tcaccctcca 3486

ggctgctgtg agagtgtcaa gtgtgtaagg gcccaaactc aggttcagtg cagaaccagg 3546

tcagcaggta tgcccgcccg taggttaagg gggccctcta aaccccttgc ctggcctcac 3606

ctggccagct cacccctttt gggtgtaggg gaaaagaatg cctgaccctg ggaaggctcc 3666

ctggtagaat acaccacact tttcaggttg ttgcaacaca ggtcctgagt tgacctctgg 3726

ttcagccaag gaccaaagaa ggtgtgtaag tgaagtggtt ctcagtcccc agacatgtgc 3786

ccctttgctg ctggctacca ctcttcccca gagcagcagg ccccgagccc cttcaggccc 3846

agcactgccc cagactcgct ggcactcagt tccctcatct gtaaaggtga agggtgatgc 3906

aggatatgcc tgacaggaac agtctgtgga tggacatgat cagtgctaag gaaagcagca 3966

gagagagacg tccggcgccc cagccccact atcagtgtcc agcgtgctgg ttccccagag 4026

cacagctcag catcacactg acactcaccc tgccctgccc ctggccagag ggtactgccg 4086

acggcacttt gcactctgat gacctcaaag cactttcatg gctgccctct ggcagggcag 4146

ggcagggcag tgacactgta ggagcatagc aagccaggag atggggtgaa gggacacagt 4206

cttgagctgt ccacatgcat gtgactcctc aaacctcttc cagatttctc taagaatagc 4266

acccccttcc ccattgcccc agcttagcct cttctcccag gggagctact caggactcac 4326

gtagcattaa atcagctgtg aatcgtcagg gggtgtctgc tagcctcaac ctcctggggc 4386

aggggacgcc gagactccgt gggagaagct cattcccaca tcttgccaag acagcctttg 4446

tccagctgtc cacattgagt cagactgctc ccggggagag agccccggcc cccagcacat 4506

aaagaactgc agccttggta ctgcagagtc tgggttgtag agaactcttt gtaagcaata 4566

aagtttgggg tgatgacaaa tgttaaaaaa 4596

<210> 2
<211> 947
<212> PRT
<213> Homo sapiens

<400> 2
Met Ala Val Met Glu Met Ala Cys Pro Gly Ala Pro Gly Ser Ala Val
1               5                   10                  15
Gly Gln Gln Lys Glu Leu Pro Lys Pro Lys Glu Lys Thr Pro Pro Leu
            20                  25                  30
Gly Lys Lys Gln Ser Ser Val Tyr Lys Leu Glu Ala Val Glu Lys Ser
        35                  40                  45
Pro Val Phe Cys Gly Lys Trp Glu Ile Leu Asn Asp Val Ile Thr Lys
    50                  55                  60
Gly Thr Ala Lys Glu Gly Ser Glu Ala Gly Pro Ala Ala Ile Ser Ile
65                  70                  75                  80
Ile Ala Gln Ala Glu Cys Glu Asn Ser Gln Glu Phe Ser Pro Thr Phe
                85                  90                  95
Ser Glu Arg Ile Phe Ile Ala Gly Ser Lys Gln Tyr Ser Gln Ser Glu
            100                 105                 110
Ser Leu Asp Gln Ile Pro Asn Asn Val Ala His Ala Thr Glu Gly Lys
        115                 120                 125
Met Ala Arg Val Cys Trp Lys Gly Lys Arg Arg Ser Lys Ala Arg Lys
    130                 135                 140
Lys Arg Lys Lys Lys Ser Ser Lys Ser Leu Ala His Ala Gly Val Ala
145                 150                 155                 160
Leu Ala Lys Pro Leu Pro Arg Thr Pro Glu Gln Glu Ser Cys Thr Ile
                165                 170                 175
Pro Val Gln Glu Asp Glu Ser Pro Leu Gly Ala Pro Tyr Val Arg Asn
            180                 185                 190
Thr Pro Gln Phe Thr Lys Pro Leu Lys Glu Pro Gly Leu Gly Gln Leu
        195                 200                 205
Cys Phe Lys Gln Leu Gly Glu Gly Leu Arg Pro Ala Leu Pro Arg Ser
    210                 215                 220
Glu Leu His Lys Leu Ile Ser Pro Leu Gln Cys Leu Asn His Val Trp
225                 230                 235                 240
Lys Leu His His Pro Gln Asp Gly Gly Pro Leu Pro Leu Pro Thr His
                245                 250                 255
Pro Phe Pro Tyr Ser Arg Leu Pro His Pro Phe Pro Phe His Pro Leu
            260                 265                 270
Gln Pro Trp Lys Pro His Pro Leu Glu Ser Phe Leu Gly Lys Leu Ala
        275                 280                 285
Cys Val Asp Ser Gln Lys Pro Leu Pro Asp Pro His Leu Ser Lys Leu
    290                 295                 300
Ala Cys Val Asp Ser Pro Lys Pro Leu Pro Gly Pro His Leu Glu Pro
305                 310                 315                 320
Ser Cys Leu Ser Arg Gly Ala His Glu Lys Phe Ser Val Glu Glu Tyr
                325                 330                 335
Leu Val His Ala Leu Gln Gly Ser Val Ser Ser Ser Gln Ala His Ser
            340                 345                 350
Leu Thr Ser Leu Ala Lys Thr Trp Ala Ala Arg Gly Ser Arg Ser Arg
        355                 360                 365
Glu Pro Ser Pro Lys Thr Glu Asp Asn Glu Gly Val Leu Leu Thr Glu
    370                 375                 380
Lys Leu Lys Pro Val Asp Tyr Glu Tyr Arg Glu Glu Val His Trp Ala
385                 390                 395                 400
Thr His Gln Leu Arg Leu Gly Arg Gly Ser Phe Gly Glu Val His Arg

                405                 410                 415
Met Glu Asp Lys Gln Thr Gly Phe Gln Cys Ala Val Lys Lys Val Arg
            420                 425                 430
Leu Glu Val Phe Arg Ala Glu Glu Leu Met Ala Cys Ala Gly Leu Thr
        435                 440                 445
Ser Pro Arg Ile Val Pro Leu Tyr Gly Ala Val Arg Glu Gly Pro Trp
    450                 455                 460
Val Asn Ile Phe Met Glu Leu Leu Glu Gly Gly Ser Leu Gly Gln Leu
465                 470                 475                 480
Val Lys Glu Gln Gly Cys Leu Pro Glu Asp Arg Ala Leu Tyr Tyr Leu
                485                 490                 495
Gly Gln Ala Leu Glu Gly Leu Glu Tyr Leu His Ser Arg Arg Ile Leu
            500                 505                 510
His Gly Asp Val Lys Ala Asp Asn Val Leu Leu Ser Ser Asp Gly Ser
        515                 520                 525
His Ala Ala Leu Cys Asp Phe Gly His Ala Val Cys Leu Gln Pro Asp
    530                 535                 540
Gly Leu Gly Lys Ser Leu Leu Thr Gly Asp Tyr Ile Pro Gly Thr Glu
545                 550                 555                 560
Thr His Met Ala Pro Glu Val Val Leu Gly Arg Ser Cys Asp Ala Lys
                565                 570                 575
Val Asp Val Trp Ser Ser Cys Cys Met Met Leu His Met Leu Asn Gly
            580                 585                 590
Cys His Pro Trp Thr Gln Phe Phe Arg Gly Pro Leu Cys Leu Lys Ile
        595                 600                 605
Ala Ser Glu Pro Pro Pro Val Arg Glu Ile Pro Pro Ser Cys Ala Pro
    610                 615                 620
Leu Thr Ala Gln Ala Ile Gln Glu Gly Leu Arg Lys Glu Pro Ile His
625                 630                 635                 640
Arg Val Ser Ala Ala Glu Leu Gly Gly Lys Val Asn Arg Ala Leu Gln
                645                 650                 655
Gln Val Gly Gly Leu Lys Ser Pro Trp Arg Gly Glu Tyr Lys Glu Pro
            660                 665                 670
Arg His Pro Pro Pro Asn Gln Ala Asn Tyr His Gln Thr Leu His Ala
        675                 680                 685
Gln Pro Arg Glu Leu Ser Pro Arg Ala Pro Gly Pro Arg Pro Ala Glu
    690                 695                 700
Glu Thr Thr Gly Arg Ala Pro Lys Leu Gln Pro Pro Leu Pro Pro Glu
705                 710                 715                 720
Pro Pro Glu Pro Asn Lys Ser Pro Pro Leu Thr Leu Ser Lys Glu Glu
                725                 730                 735
Ser Gly Met Trp Glu Pro Leu Pro Leu Ser Ser Leu Glu Pro Ala Pro
            740                 745                 750
Ala Arg Asn Pro Ser Ser Pro Glu Arg Lys Ala Thr Val Pro Glu Gln
        755                 760                 765
Glu Leu Gln Gln Leu Glu Ile Glu Leu Phe Leu Asn Ser Leu Ser Gln
    770                 775                 780
Pro Phe Ser Leu Glu Glu Gln Glu Gln Ile Leu Ser Cys Leu Ser Ile
785                 790                 795                 800
Asp Ser Leu Ser Leu Ser Asp Asp Ser Glu Lys Asn Pro Ser Lys Ala
                805                 810                 815
Ser Gln Ser Ser Arg Asp Thr Leu Ser Ser Gly Val His Ser Trp Ser
            820                 825                 830
Ser Gln Ala Glu Ala Arg Ser Ser Ser Trp Asn Met Val Leu Ala Arg
        835                 840                 845
Gly Arg Pro Thr Asp Thr Pro Ser Tyr Phe Asn Gly Val Lys Val Gln
    850                 855                 860
Ile Gln Ser Leu Asn Gly Glu His Leu His Ile Arg Glu Phe His Arg
865                 870                 875                 880
Val Lys Val Gly Asp Ile Ala Thr Gly Ile Ser Ser Gln Ile Pro Ala
                885                 890                 895
Ala Ala Phe Ser Leu Val Thr Lys Asp Gly Gln Pro Val Arg Tyr Asp
            900                 905                 910
Met Glu Val Pro Asp Ser Gly Ile Asp Leu Gln Cys Thr Leu Ala Pro

915                 920                 925
Asp Gly Ser Phe Ala Trp Ser Trp Arg Val Lys His Gly Gln Leu Glu
    930                 935                 940
Asn Arg Pro
945

SEQUENCE LISTING

<110> Axxima Pharmaceuticals AG

<120> CELLULAR KINASES INVOLVED IN CYTOMEGALOVIRUS INFECTION
      AND THEIR INHIBITION

<130> NM002756

<140>
<141>

<160> 2

<170> PatentIn Ver. 2.1

<210> 1
<211> 2030
<212> DNA
<213> Homo sapiens

<220>
<221> CDS
<222> (338)..(1294)

<400> 1
   tggctggcaa tggccttgct gacctcgagc cgggcccacg tggggacctt tggagcacag 60
   cctacgatcc tggtgcaagg ccggtggatg cagaggccag tccatatacc acccaggcct 120
   gcgaggagcg tggtccccac ccatccagcc catatgtgca agtgcccttg acagagaggc 180
   tggtcatatc catggtgacc atttatgggc cacaacaggt ccccatctgc gcagtgaacc 240
   ctgtgctgag caccttgcag acgtgatctt gcttcgtcct gcagcactgt gcggggcagg 300
   aaaatccaag aggaagaagg atctacggat atcctgc atg tcc aag cca ccc gca   355
   ccc aac ccc aca ccc ccc cgg aac ctg gac tcc cgg acc ttc atc acc   403
   att gga gac aga aac ttt gag gtg gag gct gat gac ttg gtg acc atc   451
   tca gaa ctg ggc cgt gga gcc tat ggg gtg gta gag aag gtg cgg cac   499
   gcc cag agc ggc acc atc atg gcc gtg aag cgg atc cgg gcc acc gtg   547
   aac tca cag gag cag aag cgg ctg ctc atg gac ctg gac atc aac atg 595
   cgc acg gtc gac tgt ttc tac act gtc acc ttc tac ggg gca cta ttc 643
   aga gag gga gac gtg tgg atc tgc atg gag ctc atg gac aca tcc ttg 691
   gac aag ttc tac cgg aag gtg ctg gat aaa aac atg aca att cca gag 739
   gac atc ctt ggg gag att gct gtg tct atc gtg cgg gcc ctg gag cat 787
   ctg cac agc aag ctg tcg gtg atc cac aga gat gtg aag ccc tcc aat 835
   gtc ctt atc aac aag gag ggc cat gtg aag atg tgt gac ttt ggc atc 883
   agt ggc tac ttg gtg gac tct gtg gcc aag acg atg gat gcc ggc tgc 931
   aag ccc tac atg gcc cct gag agg atc aac cca gag ctg aac cag aag 979
   ggc tac aat gtc aag tcc gac gtc tgg agc ctg ggc atc acc atg att 1027
   gag atg gcc atc ctg cgg ttc cct tac gag tcc tgg ggg acc ccg ttc 1075
   cag cag ctg aag cag gtg gtg gag gag ccg tcc ccc cag ctc cca gcc 1123
   gac cgt ttc tcc ccc gag ttt gtg gac ttc act gct cag tgc ctg agg 1171
   aag aac ccc gca gag cgt atg agc tac ctg gag ctg atg gag cac ccc 1219
   ttc ttc acc ttg cac aaa acc aag aag acg gac att gct gcc ttc gtg 1267
   aag aag atc ctg gga gaa gac tca tag gggctgggcc tcggacccca 1314
   ctccggccct ccagagcccc acagccccat ctgcgggggc agtgctcacc cacaccataa 1374
   gctactgcca tcctggccca gggcatctgg gaggaaccga gggggctgct cccacctggc 1434
   tctgtggcga gccatttgtc ccaagtgcca aagaagcaga ccattggggc tcccagccag 1494
   gcccttgtcg gccccaccag tgcctctccc tgctgctcct aggacccgtc tccagctgct 1554
   gagatcctgg actgaggggg cctggatgcc ccctgtggat gctgctgccc ctgcacagca 1614
   ggctgccagt gcctgggtgg atgggccacc gccttgccca gcctggatgc catccaagtt 1674
   gtatattttt ttaatctctc gactgaatgg actttgcaca ctttggccca gggtggccac 1734
   acctctatcc cggctttggt gcggggtaca caagagggga tgagttgtgt gaatacccca 1794
   agactcccat gagggagatg ccatgagccg cccaaggcct tcccctggca ctggcaaaca 1854
   gggcctctgc ggagcacact ggctcaccca gtcctgcccg ccaccgttat cggtgtcatt 1914
   cacctttcgt gtttttttta atttatcctc tgttgatttt ttcttttgct ttatgggttt 1974
   ggcttgtttt tcttgcatgg tttggagctg atcgcttctc ccccaccccc tagggg 2030

<210> 2
<211> 318
<212> PRT
<213> Homo sapiens

<400> 2

SEQUENCE LISTING

<110> Axxima Pharmaceuticals AG

<120> CELLULAR KINASES INVOLVED IN CYTOMEGALOVIRUS INFECTION
      AND THEIR INHIBITION

<130> U 88666

<140>
<141>

<160> 2

<170> PatentIn Ver. 2.1

<210> 1
<211> 3745
<212> DNA
<213> Homo sapiens

<220>
<221> CDS
<222> (188)..(2248)

<400> 1
gaattcggca cgaggccatt gaatcccagt cctaacagaa gtactgcgaa tcttgtggcc 60

tcattctgaa caaaagggat tagagaagaa aaatctcttg atataaggct tgaaagcaag 120

ggcaggcaat cttggttgtg aatattttct gatttttcca gaaatcaagc agaagattga 180

gctgctg atg tca gtt aac tct gag aag tcg tcc tct tca gaa agg ccg   229
        Met Ser Val Asn Ser Glu Lys Ser Ser Ser Ser Glu Arg Pro
          1               5                  10

gag cct caa cag aaa gct cct tta gtt cct cct cct cca ccg cca cca   277
Glu Pro Gln Gln Lys Ala Pro Leu Val Pro Pro Pro Pro Pro Pro Pro
 15                  20                  25                  30

cca cca cca ccg cca cct ttg cca gac ccc aca ccc ccg gag cca gag   325
Pro Pro Pro Pro Pro Pro Leu Pro Asp Pro Thr Pro Pro Glu Pro Glu
                 35                  40                  45

gag gag atc ctg gga tca gat gat gag gag caa gag gac cct gcg gac   373
Glu Glu Ile Leu Gly Ser Asp Asp Glu Glu Gln Glu Asp Pro Ala Asp
             50                  55                  60

tac tgc aaa ggt gga tat cat cca gtg aaa att gga gac ctc ttc aat   421
Tyr Cys Lys Gly Gly Tyr His Pro Val Lys Ile Gly Asp Leu Phe Asn
         65                  70                  75

ggc cgg tat cat gtt att aga aag ctt gga tgg ggg cac ttc tct act   469
Gly Arg Tyr His Val Ile Arg Lys Leu Gly Trp Gly His Phe Ser Thr
     80                  85                  90

gtc tgg ctg tgc tgg gat atg cag ggg aaa aga ttt gtt gca atg aaa    517
Val Trp Leu Cys Trp Asp Met Gln Gly Lys Arg Phe Val Ala Met Lys
 95                 100                 105                 110

gtt gta aaa agt gcc cag cat tat acg gag aca gcc ttg gat gaa ata    565
Val Val Lys Ser Ala Gln His Tyr Thr Glu Thr Ala Leu Asp Glu Ile
                115                 120                 125

aaa ttg ctc aaa tgt gtt cga gaa agt gat ccc agt gac cca aac aaa    613
Lys Leu Leu Lys Cys Val Arg Glu Ser Asp Pro Ser Asp Pro Asn Lys
            130                 135                 140

gac atg gtg gtc cag ctc att gac gac ttc aag att tca ggc atg aat    661
Asp Met Val Val Gln Leu Ile Asp Asp Phe Lys Ile Ser Gly Met Asn
        145                 150                 155

ggg ata cat gtc tgc atg gtc ttc gaa gta ctt ggc cac cat ctc ctc    709
Gly Ile His Val Cys Met Val Phe Glu Val Leu Gly His His Leu Leu
    160                 165                 170

aag tgg atc atc aaa tcc aac tat caa ggc ctc cca gta cgt tgt gtg    757
Lys Trp Ile Ile Lys Ser Asn Tyr Gln Gly Leu Pro Val Arg Cys Val
175                 180                 185                 190

aag agt atc att cga cag gtc ctt caa ggg tta gat tac tta cac agt    805
Lys Ser Ile Ile Arg Gln Val Leu Gln Gly Leu Asp Tyr Leu His Ser
                195                 200                 205

aag tgc aag atc att cat act gac ata aag ccg gaa aat atc ttg atg    853
Lys Cys Lys Ile Ile His Thr Asp Ile Lys Pro Glu Asn Ile Leu Met
            210                 215                 220

tgt gtg gat gat gca tat gtg aga aga atg gca gct gag cct gag tgg    901
Cys Val Asp Asp Ala Tyr Val Arg Arg Met Ala Ala Glu Pro Glu Trp
        225                 230                 235

cag aaa gca ggt gct cct cct cct tca ggg tct gca gtg agt acg gct    949
Gln Lys Ala Gly Ala Pro Pro Pro Ser Gly Ser Ala Val Ser Thr Ala
    240                 245                 250

cca cag cag aaa cct ata gga aaa ata tct aaa aac aaa aag aaa aaa    997
Pro Gln Gln Lys Pro Ile Gly Lys Ile Ser Lys Asn Lys Lys Lys Lys
255                 260                 265                 270

ctg aaa aag aaa cag aag agg cag gct gag tta ttg gag aag cgc ctg    1045
Leu Lys Lys Lys Gln Lys Arg Gln Ala Glu Leu Leu Glu Lys Arg Leu
                275                 280                 285

cag gag ata gaa gaa ttg gag cga gaa gct gaa agg aaa ata ata gaa    1093
Gln Glu Ile Glu Glu Leu Glu Arg Glu Ala Glu Arg Lys Ile Ile Glu
            290                 295                 300

gaa aac atc acc tca gct gca cct tcc aat gac cag gat ggc gaa tac    1141
Glu Asn Ile Thr Ser Ala Ala Pro Ser Asn Asp Gln Asp Gly Glu Tyr
        305                 310                 315

tgc cca gag gtg aaa cta aaa aca aca gga tta gag gag gcg gct gag    1189
Cys Pro Glu Val Lys Leu Lys Thr Thr Gly Leu Glu Glu Ala Ala Glu
    320                 325                 330

gca gag act gca aag gac aat ggt gaa gct gag gac cag gaa gag aaa    1237
Ala Glu Thr Ala Lys Asp Asn Gly Glu Ala Glu Asp Gln Glu Glu Lys
335                 340                 345                 350

gaa gat gct gag aaa gaa aac att gaa aaa gat gaa gat gat gta gat 1285
Glu Asp Ala Glu Lys Glu Asn Ile Glu Lys Asp Glu Asp Asp Val Asp
                355                 360                 365

cag gaa ctt gcg aac ata gac cct acg tgg ata gaa tca cct aaa acc 1333
Gln Glu Leu Ala Asn Ile Asp Pro Thr Trp Ile Glu Ser Pro Lys Thr
            370                 375                 380

aat ggc cat att gag aat ggc cca ttc tca ctg gag cag caa ctg gac 1381
Asn Gly His Ile Glu Asn Gly Pro Phe Ser Leu Glu Gln Gln Leu Asp
        385                 390                 395

gat gaa gat gat gat gaa gaa gac tgc cca aat cct gag gaa tat aat 1429
Asp Glu Asp Asp Asp Glu Glu Asp Cys Pro Asn Pro Glu Glu Tyr Asn
    400                 405                 410

ctt gat gag cca aat gca gaa agt gat tac aca tat agc agc tcc tat 1477
Leu Asp Glu Pro Asn Ala Glu Ser Asp Tyr Thr Tyr Ser Ser Ser Tyr
415                 420                 425                 430

gaa caa ttc aat ggt gaa ttg cca aat gga cga cat aaa att ccc gag 1525
Glu Gln Phe Asn Gly Glu Leu Pro Asn Gly Arg His Lys Ile Pro Glu
                435                 440                 445

tca cag ttc cca gag ttt tcc acc tcg ttg ttc tct gga tcc tta gaa 1573
Ser Gln Phe Pro Glu Phe Ser Thr Ser Leu Phe Ser Gly Ser Leu Glu
            450                 455                 460

cct gtg gcc tgc ggc tct gtg ctt tct gag gga tca cca ctt act gag 1621
Pro Val Ala Cys Gly Ser Val Leu Ser Glu Gly Ser Pro Leu Thr Glu
        465                 470                 475

caa gag gag agc agt cca tcc cat gac aga agc aga acg gtt tca gcc 1669
Gln Glu Glu Ser Ser Pro Ser His Asp Arg Ser Arg Thr Val Ser Ala
    480                 485                 490

tcc agt act ggg gat ttg cca aaa gca aaa acc cgg gca gct gac ttg 1717
Ser Ser Thr Gly Asp Leu Pro Lys Ala Lys Thr Arg Ala Ala Asp Leu
495                 500                 505                 510

ttg gtg aat ccc ctg gat ccg cgg aat cga gat aaa att aga gta aaa 1765
Leu Val Asn Pro Leu Asp Pro Arg Asn Arg Asp Lys Ile Arg Val Lys
                515                 520                 525

att gct gac ctg gga aat gct tgt tgg gtg cat aaa cac ttc acg gaa 1813
Ile Ala Asp Leu Gly Asn Ala Cys Trp Val His Lys His Phe Thr Glu
            530                 535                 540

gac atc cag acg cgt cag tac cgc tcc ata gag gtt tta ata gga gcg 1861
Asp Ile Gln Thr Arg Gln Tyr Arg Ser Ile Glu Val Leu Ile Gly Ala
        545                 550                 555

ggg tac agc acc cct gcg gac atc tgg agc acg gcg tgt atg gca ttt 1909
Gly Tyr Ser Thr Pro Ala Asp Ile Trp Ser Thr Ala Cys Met Ala Phe
    560                 565                 570

gag ctg gca acg gga gat tat ttg ttt gaa cca cat tct ggg gaa gac 1957
Glu Leu Ala Thr Gly Asp Tyr Leu Phe Glu Pro His Ser Gly Glu Asp
575                 580                 585                 590

tat tcc aga gac gaa gac cac ata gcc cac atc ata gag ctg cta ggc 2005
Tyr Ser Arg Asp Glu Asp His Ile Ala His Ile Ile Glu Leu Leu Gly
                595                 600                 605

agt att cca agg cac ttt gct cta tct gga aaa tat tct cgg gaa ttc    2053
Ser Ile Pro Arg His Phe Ala Leu Ser Gly Lys Tyr Ser Arg Glu Phe
            610                 615                 620

ttc aat cgc aga gga gaa ctg cga cac atc acc aag ctg aag ccc tgg    2101
Phe Asn Arg Arg Gly Glu Leu Arg His Ile Thr Lys Leu Lys Pro Trp
        625                 630                 635

agc ctc ttt gat gta ctt gtg gaa aag tat ggc tgg ccc cat gaa gat    2149
Ser Leu Phe Asp Val Leu Val Glu Lys Tyr Gly Trp Pro His Glu Asp
    640                 645                 650

gct gca cag ttt aca gat ttc ctg atc ccg atg tta gaa atg gtt cca    2197
Ala Ala Gln Phe Thr Asp Phe Leu Ile Pro Met Leu Glu Met Val Pro
655                 660                 665                 670

gaa aaa cga gcc tca gct ggc gaa tgt cgg cat cct tgg ttg aat tct    2245
Glu Lys Arg Ala Ser Ala Gly Glu Cys Arg His Pro Trp Leu Asn Ser
                675                 680                 685

tag caaattctac caatattgca ttctgagcta gcaaatgttc ccagtacatt    2298

ggacctaaac ggtgactctc attctttaac aggattacaa gtgagctggc ttcatcctca 2358

gacctttatt ttgctttgag gtactgttgt ttgacatttt gctttttgtg cactgtgatc 2418

ctggggaagg gtagtctttt gtcttcagct aagtagttta ctgaccattt tcttctggaa 2478

acaataacat gtctctaagc attgtttctt gtgttgtgtg acattcaaat gtcatttttt 2538

tgaatgaaaa atactttccc ctttgtgttt tggcaggttt tgtaactatt tatgaagaaa 2598

tattttagct gagtactata taatttacaa tcttaagaaa ttatcaagtt ggaaccaaga 2658

aatagcaagg aaatgtacaa ttttatcttc tggcaaaggg acatcattcc tgtattatag 2718

tgtatgtaaa tgcaccctgt aaatgttact ttccattaaa tatgggaggg ggactcaaat 2778

ttcagaaaag ctaccaagtc ttgagtgctt tgtagcctat gttgcatgta gcggacttta 2838

actgctccaa ggagttgtgc aaacttttca ttccataaca gtcttttcac attggatttt 2898

aaacaaagtg gctctgggtt ataagatgtc attctctata tggcacttta aaggaagaaa 2958

agatatgttt ctcattctaa aatatgcatt ataatttagc agtcccattt gtgattttgc 3018

atatttttaa aagtactttt aaagaagagc aatttccctt taaaaatgtg atggctcagt 3078

accatgtcat gttgcctcct ctgggcgctg taagttaagc tctacataga ttaaattgga 3138

gaaacgtgtt aattgtgtgg aatgaaaaaa tacatatatt tttggaaaag catgatcatg 3198

cttgtctaga acacaaggta tggtatatac aatttgcagt gcagtgggca gaatacttct 3258

cacagctcaa agataacagt gatcacattc attccatagg tagctttacg tgtggctaca 3318

acaaatttta ctagcttttt cattgtcttt ccatgaaacg aagttgagaa aatgattttc 3378

cctttgcagg ttgcacacag ttttgtttat gcatttcctt aaaattaatt gtagactcca 3438

ggatacaaac catagtaggc aatacaattt agaatgtaat atatagaggt atattagcct 3498

ctttagaagt cagtggattg aatgtctttt tattttaaat tttacattca ttaaggtgcc 3558

tcgtttttga ctttgtccat taacatttat ccatatgcct ttgcaataac tagattgtga 3618

aaagctaaca agtgttgtaa caataatcca ttgtttgagg tgcttgcagt tgtcttaaaa 3678

attaaagtgt tttggttttt ttttttccag aaaaaaaaaa aaaaaaaaaa aaaaaaaaaa 3738

ttcctgc 3745

<210> 2
<211> 686
<212> PRT
<213> Homo sapiens

<400> 2
Met Ser Val Asn Ser Glu Lys Ser Ser Ser Ser Glu Arg Pro Glu Pro
1               5                   10                  15
Gln Gln Lys Ala Pro Leu Val Pro Pro Pro Pro Pro Pro Pro Pro Pro
            20                  25                  30
Pro Pro Pro Pro Leu Pro Asp Pro Thr Pro Pro Glu Pro Glu Glu Glu
        35                  40                  45
Ile Leu Gly Ser Asp Asp Glu Glu Gln Glu Asp Pro Ala Asp Tyr Cys
    50                  55                  60
Lys Gly Gly Tyr His Pro Val Lys Ile Gly Asp Leu Phe Asn Gly Arg
65                  70                  75                  80
Tyr His Val Ile Arg Lys Leu Gly Trp Gly His Phe Ser Thr Val Trp
                85                  90                  95
Leu Cys Trp Asp Met Gln Gly Lys Arg Phe Val Ala Met Lys Val Val
            100                 105                 110
Lys Ser Ala Gln His Tyr Thr Glu Thr Ala Leu Asp Glu Ile Lys Leu
        115                 120                 125
Leu Lys Cys Val Arg Glu Ser Asp Pro Ser Asp Pro Asn Lys Asp Met
    130                 135                 140
Val Val Gln Leu Ile Asp Asp Phe Lys Ile Ser Gly Met Asn Gly Ile
145                 150                 155                 160
His Val Cys Met Val Phe Glu Val Leu Gly His His Leu Leu Lys Trp
                165                 170                 175
Ile Ile Lys Ser Asn Tyr Gln Gly Leu Pro Val Arg Cys Val Lys Ser
            180                 185                 190
Ile Ile Arg Gln Val Leu Gln Gly Leu Asp Tyr Leu His Ser Lys Cys
        195                 200                 205
Lys Ile Ile His Thr Asp Ile Lys Pro Glu Asn Ile Leu Met Cys Val
    210                 215                 220
Asp Asp Ala Tyr Val Arg Arg Met Ala Ala Glu Pro Glu Trp Gln Lys
225                 230                 235                 240
Ala Gly Ala Pro Pro Pro Ser Gly Ser Ala Val Ser Thr Ala Pro Gln
                245                 250                 255
Gln Lys Pro Ile Gly Lys Ile Ser Lys Asn Lys Lys Lys Lys Leu Lys
            260                 265                 270
Lys Lys Gln Lys Arg Gln Ala Glu Leu Leu Glu Lys Arg Leu Gln Glu
        275                 280                 285
Ile Glu Glu Leu Glu Arg Glu Ala Glu Arg Lys Ile Ile Glu Glu Asn
    290                 295                 300
Ile Thr Ser Ala Ala Pro Ser Asn Asp Gln Asp Gly Glu Tyr Cys Pro
305                 310                 315                 320
Glu Val Lys Leu Lys Thr Thr Gly Leu Glu Glu Ala Ala Glu Ala Glu
                325                 330                 335
Thr Ala Lys Asp Asn Gly Glu Ala Glu Asp Gln Glu Glu Lys Glu Asp
            340                 345                 350
Ala Glu Lys Glu Asn Ile Glu Lys Asp Glu Asp Asp Val Asp Gln Glu
        355                 360                 365
Leu Ala Asn Ile Asp Pro Thr Trp Ile Glu Ser Pro Lys Thr Asn Gly

370                 375                 380
His Ile Glu Asn Gly Pro Phe Ser Leu Glu Gln Gln Leu Asp Asp Glu
385                 390                 395                 400
Asp Asp Asp Glu Glu Asp Cys Pro Asn Pro Glu Glu Tyr Asn Leu Asp
                405                 410                 415
Glu Pro Asn Ala Glu Ser Asp Tyr Thr Tyr Ser Ser Ser Tyr Glu Gln
            420                 425                 430
Phe Asn Gly Glu Leu Pro Asn Gly Arg His Lys Ile Pro Glu Ser Gln
        435                 440                 445
Phe Pro Glu Phe Ser Thr Ser Leu Phe Ser Gly Ser Leu Glu Pro Val
    450                 455                 460
Ala Cys Gly Ser Val Leu Ser Glu Gly Ser Pro Leu Thr Glu Gln Glu
465                 470                 475                 480
Glu Ser Ser Pro Ser His Asp Arg Ser Arg Thr Val Ser Ala Ser Ser
                485                 490                 495
Thr Gly Asp Leu Pro Lys Ala Lys Thr Arg Ala Ala Asp Leu Leu Val
            500                 505                 510
Asn Pro Leu Asp Pro Arg Asn Arg Asp Lys Ile Arg Val Lys Ile Ala
        515                 520                 525
Asp Leu Gly Asn Ala Cys Trp Val His Lys His Phe Thr Glu Asp Ile
    530                 535                 540
Gln Thr Arg Gln Tyr Arg Ser Ile Glu Val Leu Ile Gly Ala Gly Tyr
545                 550                 555                 560
Ser Thr Pro Ala Asp Ile Trp Ser Thr Ala Cys Met Ala Phe Glu Leu
                565                 570                 575
Ala Thr Gly Asp Tyr Leu Phe Glu Pro His Ser Gly Glu Asp Tyr Ser
            580                 585                 590
Arg Asp Glu Asp His Ile Ala His Ile Ile Glu Leu Leu Gly Ser Ile
        595                 600                 605
Pro Arg His Phe Ala Leu Ser Gly Lys Tyr Ser Arg Glu Phe Phe Asn
    610                 615                 620
Arg Arg Gly Glu Leu Arg His Ile Thr Lys Leu Lys Pro Trp Ser Leu
625                 630                 635                 640
Phe Asp Val Leu Val Glu Lys Tyr Gly Trp Pro His Glu Asp Ala Ala
                645                 650                 655
Gln Phe Thr Asp Phe Leu Ile Pro Met Leu Glu Met Val Pro Glu Lys
            660                 665                 670
Arg Ala Ser Ala Gly Glu Cys Arg His Pro Trp Leu Asn Ser
        675                 680                 685

## Description

The present invention is in the fields of molecular biology and virology. The present invention is directed to novel methods for treating Cytomegalovirus using kinase inhibitors.

### Background of the invention

Human Cytomegalovirus (HCMV) is a highly specific β-herpesvirus. Primary infection of healthy children and adults is usually asymptomatic, with a minority of cases developing a mononucleose-like syndrome. In contrast, congenital infection (U.S. 0.2%-2.2% per live birth; aprox. 40,000 per year) leads to several neurological defects in 10-15% of infected neonates. Immunocompromised patients represent another host group facing serious disease complications caused by HCMV infection or reactivation of a persistent infection. Up to 40% of the AIDS patients, for example, develop retinitis, pneumonitis, gastroenteritis or disseminated HCMV disease. In addition, allograft recipients (20,000 allograft transplantations per year in the U.S.) are often infected (or superinfected) by virus from the transplanted organ.

Clinical symptoms in the posttransplant period include prolonged fever, leukopenia, thrombocytopenia, atypical lymphocytosis, elevated hepatic transaminases and decreased graft survival. In bone marrow transplantations, HCMV infection is associated with high mortality rates (80-90% for untreated HCMV pneumonia).

Current approaches to develop therapeutics against Cytomegalovirus (CMV) have focused on antiviral agents *per se;* for example viral polymerase inhibitors. In fact, high mortality rates have been dramatically reduced by new antiviral agents. Current CMV therapeutics possess severe drawbacks, however. For example, Fomivirsen (Vitravene, formerly ISIS 2922) is typically administered by injection directly into the eye every 2 or 4 weeks. Ganciclovir is available for intravenous (Cytovene) or oral administration, and as an implant in the case of retinitis; unfortunately, toxic complications including leukopenia and thrombocytopenia frequently develop. Foscarnet (Foscavir; phosphonoformic acid), another antiviral agent, exhibits considerable renal toxicities and is only available in intravenous form (which is also true for Cidofovir (Vistide), another CMV therapeutic). In addition, CMV replication resumes soon after Ganciclovir and Foscarnet treatment is halted. Finally, Ganciclovir- and Foscarnet-resistant strains of CMV are emerging.

Although treatment of HCMV-induced disease has been improved with these inhibitors of the viral polymerase and preemptive or early antiviral therapy in transplant patients, there is a need in the art for a new class of HCMV therapeutics with better oral bioavailability and reduced toxic effects. This is especially true in the treatment of retinitis in AIDS patients, where CMV infection must be controlled for long periods of time.

Recent research has revealed how cells communicate with each other to coordinate the growth and maintenance of the multitude of tissues within the human body. A key element of this communication network is the transmission of a signal from the exterior of a cell to its nucleus, which results in the activation or suppression of specific genes. This process is called signal transduction.

An integral part of signal transduction is the interaction of cytokines, their receptors, and intracellular signal transduction molecules. Cytokines serve as messengers that bind to receptors on the surface of a target cell. As a result of the binding, the receptors activate a cascade of downstream signaling molecules, thereby transmitting the message from the exterior of the cell to its nucleus. Signal transduction to the nucleus modulates specific gene expression (i.e., transcription and translation), which results in either the upregulation or downregulation of specific proteins that carry out a particular biological function.

Viral infection disrupts normal signal transduction, which leads to cellular malfunctioning resulting in a disease state. Specifically, interference of HCMV with relevant human primary cells is necessary for the virus to create an environment that allows it to grow and replicate, and in turn cause disease in the infected individual. Current research efforts have failed to elucidate all the specific intracellular signal pathways affected by HCMV infection, however. Discovery of the signal transduction pathways and specific intracellular signal transduction molecules affected by CMV infection would represent a tremendous advance in the understanding of the induction and progression of CMV infection processes and provide new avenues for the development of a novel class of effective therapeutics for the treatment of CMV.

Thus, object of the present invention is to provide methods for detecting, preventing and/or treating Cytomegalovirus infection and/or associated diseases, methods for the identification of compounds useful for preventing and/or treating Cytomegalovirus infection and/or associated diseases and for regulating the production of Cytomegaloviruses.

The object of the present invention is solved by the teaching of the independent claims. Further advantageous features, aspects and details of the invention are evident from the dependent claims, the description, and the examples of the present application.

### Description of the invention

The present invention is based upon the finding of a group of cellular kinases that are specifically upregulated as a result of CMV infection. The antiviral therapeutic research approach described herein, focuses on discovering the cellular signal transduction pathways involved in viral infection. Identification of the cellular signal transduction molecules, key to viral infection provides for, among other things, novel diagnostic methods, especially assays, and compositions useful therefor, novel targets for antiviral therapeutics, a novel class of antiviral therapeutics, and new screening assays and materials to discover new antiviral agents.

This approach led to the development of a novel microarray platform technology, wherein a microarray of more than 1100 signal transduction cDNAs was developed. This unique microarray technology was used to identify RNA expression patterns (e.g., upregulation or downregulation) unique to CMV infected host cells. Differential display techniques were used to pinpoint those signal transduction molecules useful as targets for drug intervention. Effective manipulation of these virally-controlled intracellular signal transduction pathways can alter (slow or stop altogether) the course of viral growth.

It is now revealed for the first time that the cellular protein kinases RICK (also known as CARDIAK; RIP2), RIP, NIK (also known as HGK; MAP4K4), MKK3 (also known as MEK3), and SRPK-2 are specifically and uniquely upregulated in a cell as a result of CMV infection. These cellular kinases therefore identify novel diagnostic and therapeutic targets for CMV infection.

Surprisingly, it was found that the following human cellular targets are significantly upregulated compared with uninfected human foreskin fibroblasts cells:

| target | upregulation |
|---|---|
| RICK | 3.6 fold |
| RIP | 2.6 fold |
| NIK | 4.0 fold |
| MKK3 | 2.5 fold |
| SRPK-2 | 2.2 fold |

Based upon the research work reported herein, one aspect of the present invention is directed to a method, preferably a screening assay, for identifying compounds useful for treating and/or preventing Cytomegalovirus infection and/or diseases associated therewith. Specifically, this assay involves contacting a test compound with one or more cellular kinases selected from the group consisting of RICK, RIP, NIK, MKK3, and SRPK-2, and detecting a change, normally a decrease, in activity of said cellular kinase. This method was used for the identification of the RICK and RIP inhibitors shown below in Table 1 and Table 2.

Another aspect of the invention is directed to a diagnostic method for detecting Cytomegalovirus infection and/or associated diseases in an individual or in cells and/or in cell lysates. This assay involves providing a sample from the individual or providing a sample from said cells, respectively, and detecting activity of one or more cellular kinases selected from the group consisting of RICK, RIP, NIK, MKK3, and SRPK-2. The term "individual" preferably refers to mammals, especially humans or ruminants.

Also described in the present invention are monoclonal or polyclonal antibodies which bind to a cellular kinase selected from the group consisting of RICK, RIP, NIK, MKK3, and SRPK-2.

A further aspect of the present invention relates to a method for preventing and/or treating Cytomegalovirus infection and/or associated diseases in an individual by administering a pharmaceutically effective amount of an inhibitor to said individual, wherein said inhibitor inhibits at least partially the activity of one or more cellular kinases selected from the group consisting of RICK, RIP, NIK, MKK3, and SRPK-2, or wherein said inhibitor inhibits at least partially the production of one or more cellular kinases selected from the group consisting of RICK, RIP, NIK, MKK3, and SRPK-2.

As used herein, the term "inhibitor" refers to any compound capable of downregulating, decreasing, reducing, suppressing or inactivating the amount and/or activity of at least one human cellular protein kinase selected from the group consisting of RICK, RIP, NIK, MKK3, and SRPK-2. Generally, said inhibitors, including suicide inhibitors, may be proteins, oligo- and polypeptides, nucleic acids, genes, small chemical molecules, or other chemical moieties. Suitable inhibitors are monoclonal or polyclonal antibodies which bind to at least one cellular kinase selected from the group consisting of RICK, RIP, NIK, MKK3, and SRPK-2.

Based on the surprising results reported herein, one aspect of the present invention is directed to a method for regulating the production of Cytomegalovirus in an individual by administering an individual a pharmaceutically effective amount of an inhibitor wherein said inhibitor inhibits at least partially the activity of one or more cellular kinases selected from the group consisting of RICK, RIP, NIK, MKK3, and SRPK-2, or wherein said inhibitor at least partially inhibits the production of one or more cellular kinases selected from the group consisting of RICK, RIP, NIK, MKK3, and SRPK-2.

A similar aspect relates to a method for regulating the production of Cytomegalovirus in cells by administering the cells a pharmaceutically effective amount of an inhibitor wherein said inhibitor inhibits at least partially the activity of one or more cellular kinases selected from the group consisting of RICK, RIP, NIK, MKK3, and SRPK-2, or wherein said inhibitor at least partially inhibits the production of one or more cellular kinases selected from the group consisting of RICK, RIP, NIK, MKK3, and SRPK-2 in the cells.

Yet another aspect of the invention is directed to a method for regulating the expression of at least one cellular kinase selected from the group consisting of RICK, RIP, NIK, MKK3, and SRPK-2 in an individual comprising the step of administering the individual a pharmaceutically effective amount of an inhibitor wherein said inhibitor inhibits at least partially the transcription of DNA or the translation of RNA encoding one of said cellular kinases.

A further aspect relates to a method for regulating the expression of at least one cellular kinase selected from the group consisting of RICK, RIP, NIK, MKK3, and SRPK-2 in the cells comprising the step of administering the cells a pharmaceutically effective amount of an inhibitor wherein said inhibitor inhibits at least partially the transcription of DNA or the translation of RNA encoding one of said cellular kinases.

As used herein, the term "regulating expression and/or activity" generally refers to any process that functions to control or modulate the quantity or activity (functionality) of a cellular component. Static regulation maintains expression and/or activity at some given level. Upregulation refers to a relative increase in expression and/or activity. Accordingly downregulation refers to a relative decrease in expression and/or activity. In the present invention, regulation is preferably the downregulation of a cellular component. Downregulation is synonymous with inhibition of a given cellular component's activity.

Beside inhibitors also activators may be useful for treating Cytomegalovirus infection by increasing the activity of at least one of the cellular protein kinases RICK, RIP, NIK, MKK3, and SRPK-2. Thus, a method for preventing and/or treating Cytomegalovirus infection and/or associated diseases in an individual is disclosed. Said method comprises administering a pharmaceutically effective amount of an activator to an individual, wherein said activator activates at least partially the activity of one or more cellular kinases selected from the group consisting of RICK, RIP, NIK, MKK3, and SRPK-2, or wherein said activator activates or stimulates at least partially the production of one or more cellular kinases selected from the group consisting of RICK, RIP, NIK, MKK3, and SRPK-2.

Furthermore, a method for regulating the production of Cytomegalovirus either in cells or in an individual is described. Said methods comprise administering an individual or to cells a pharmaceutically effective amount of an activator wherein said activator activates at least partially the activity of one or more cellular kinases selected from the group consisting of RICK, RIP, NIK, MKK3, and SRPK-2, or wherein said activator at least partially activates or stimulates the production of one or more cellular kinases selected from the group consisting of RICK, RIP, NIK, MKK3, and SRPK-2.

As used herein, the term "activator" refers to any chemical compound which is able to upregulate, increase, activate, or stimulate the activity of at least one human cellular protein kinase selected from the group consisting of RICK, RIP, NIK, MKK3, and SRPK-2 or which is able to upregulate, increase, activate, or stimulate the expression of at least one of said cellular kinases. Activators comprise proteins, oligo- and polypeptides, nucleic acids, genes, and preferably small chemical molecules, or other chemical moieties.

Still another aspect of the present invention is directed to either a method for regulating the expression of at least one cellular kinase selected from the group consisting of RICK, RIP, NIK, MKK3, and SRPK-2 in an individual or for regulating the expression of at least one of said kinases in cells. These methods comprise the step of administering the individual or the cells a pharmaceutically effective amount of an activator wherein said activator activates at least partially the transcription of DNA or the translation of RNA encoding one of said cellular kinases.

Furthermore, oligonucleotides are disclosed which bind to the DNA or RNA encoding a cellular kinase selected from the group consisting of RICK, RIP, NIK, MKK3, and SRPK-2. Said oligonucleotides can be used as suitable inhibitors within the aforementioned methods.

Some methods of the present invention identify compounds useful for prophylaxis and/or treatment of Cytomegalovirus infection and/or associated disease by screening a test compound, or a library of test compounds, for its ability to inhibit at least one of the above-mentioned human cellular protein kinases identified herein as characteristically upregulated during HCMV replication. Using this method the compounds A to E have been identified as RICK inhibitors and the compounds F to H have been identified as RIP inhibitors. Thus, the use of these compounds as inhibitors of RICK or RIP is disclosed. Furthermore, these compounds can be used for manufacturing a pharmaceutical composition for prophylaxis and/or treatment of Cytomegalovirus infection and/or diseases associated with Cytomegalovirus infection.

A variety of assay protocols and detection techniques are well known in the art and easily adapted for this purpose by a skilled practitioner. Such methods include, but are not limited to, high throughput assays (e.g., microarray technology, phage display technology), and *in vitro* and *in vivo* cellular and tissue assays.

Thus, some embodiments of the present invention may comprise a solid support useful for detecting Cytomegalovirus infection in a cell or an individual. Preferably the solid support comprises immobilized oligonucleotides, wherein the oligonucleotides are capable of detecting activity of one or more cellular kinases selected from the group consisting of: RICK, RIP, NIK, MKK3, and SRPK-2.

Another aspect of the invention includes a solid support useful for screening compounds useful for treating Cytomegalovirus. Preferred embodiments include a solid support comprising one or more immobilized oligonucleotides, wherein the oligonucleotide(s) encode one or more cellular kinases selected from the group consisting of RICK, RIP, NIK, MKK3, and SRPK-2. In another preferred embodiment, the solid support comprises one or more immobilized cellular kinases selected from the group consisting of: RICK, RIP, NIK, MKK3, and SRPK-2.

Accordingly, another aspect of the present invention is directed to a novel therapeutic composition useful to treat an individual afflicted with Cytomegalovirus comprising one or more inhibitors capable of inhibiting activity of one or more of the cellular kinases selected from the group consisting of RICK, RIP, NIK, MKK3, and SRPK-2. In addition thereto, a novel pharmaceutical composition could comprise at least one inhibitor capable of regulating the production of HCMV by inhibiting the expression of at least one cellular kinase selected from the group consisting of RICK, RIP, NIK, MKK3, and SRPK-2.

Another group of suitable therapeutic compositions useful for prophylaxis and/or treatment of CMV comprises at least one activator which is able to increase the activity of one or more cellular kinases selected from the group consisting of RICK, RIP, NIK, MKK3, and SRPK-2 or which is capable of increasing the expression of at least one cellular kinase selected from the group consisting of RICK, RIP, NIK, MKK3, and SRPK-2.

Said pharmaceutical compositions may further comprise pharmaceutically acceptable carriers, excipient, diluents, fillers, binders, disintegrants, lubricants, glidents, coloring agents, flavoring agents, opaquing agents, and/or plasticizers.

### Detailed description of the invention

Utilizing microarray technology, a unique microarray of more than 1100 signal transduction cDNAs was developed. This array was used to compare signal transduction mRNA expression patterns (e.g., upregulation or downregulation) in primary human cells before and after infection with HCMV at various timepoints of infection. Interference of the HCMV with the cellular signaling events is reflected in differential gene expression when compared to the uninfected cellular signaling. Results from this novel signal transduction microarray analysis revealed significant upregulation of cellular protein kinases RICK, RIP, NIK, MKK3, and SRPK-2, as unique to CMV infected host cells. These findings were confirmed utilizing conventional Northern and Western blot analyses.

Disclosed herein is the first report describing the role of cellular kinases; RICK, RIP, NIK, MKK3, and SRPK-2 in the signal transduction of CMV viral infection process. As a result of these discoveries, a novel class of compounds, i.e., RICK, RIP, NIK, MKK3, and SRPK-2 inhibitors, are identified as useful for altering the course of CMV infection.

To perform initial tests for compounds that inhibit RICK activity in a cellular assay, RICK was transiently overexpressed in HEK-293 cells, immunoprecipitated and incubated with different concentrations of test compounds before in-vitro kinase assays were performed (Example 10). According to the method for identifying compounds useful for inhibiting the cellular kinase RICK and therefore useful for treating and/or preventing Cytomegalovirus infection and/or diseases associated with Cytomegalovirus infection, a test compound is contacted with the cellular kinase RICK according to the RICK assay protocoll disclosed in example 10. The test compound dissolved in DMSO is added to the RICK assay solution at concentrations between 100 nM and 50 µM. Thereafter, radioactively labeled ATP is added and kinase activity of RICK is determined by detecting the autophosphorylation of RICK via radioactivity measurement. The five compounds listed in the following Table 1 were identified using said method. These compounds showed inhibition of RICK kinase activity with an IC₅₀ between about 500 nM and 1 µM and an inhibition of HCMV with an IC₅₀ between about 1 and 8 µM, respectively. The IC₅₀ values of HCMV inhibition were obtained by the use of at least one assay protocol selected from a) virus replication assay, b) plaque assay, c) GFP (Green Fluorescent Protein) infection assay, and d) indirect immunofluorescence analysis as disclosed in example 12. Thus, the five compounds A to E mentioned below and/or pharmaceutically acceptable salts thereof can be used as inhibitors of the cellular protein kinase RICK and as pharmaceutically active compounds for the treatment and/or prophylaxis of HCMV infection. Furthermore, these compounds are suitable for the manufacture of a pharmaceutical composition for prophylaxis and/or treatment of Cytomegalovirus infection and/or diseases associated therewith.

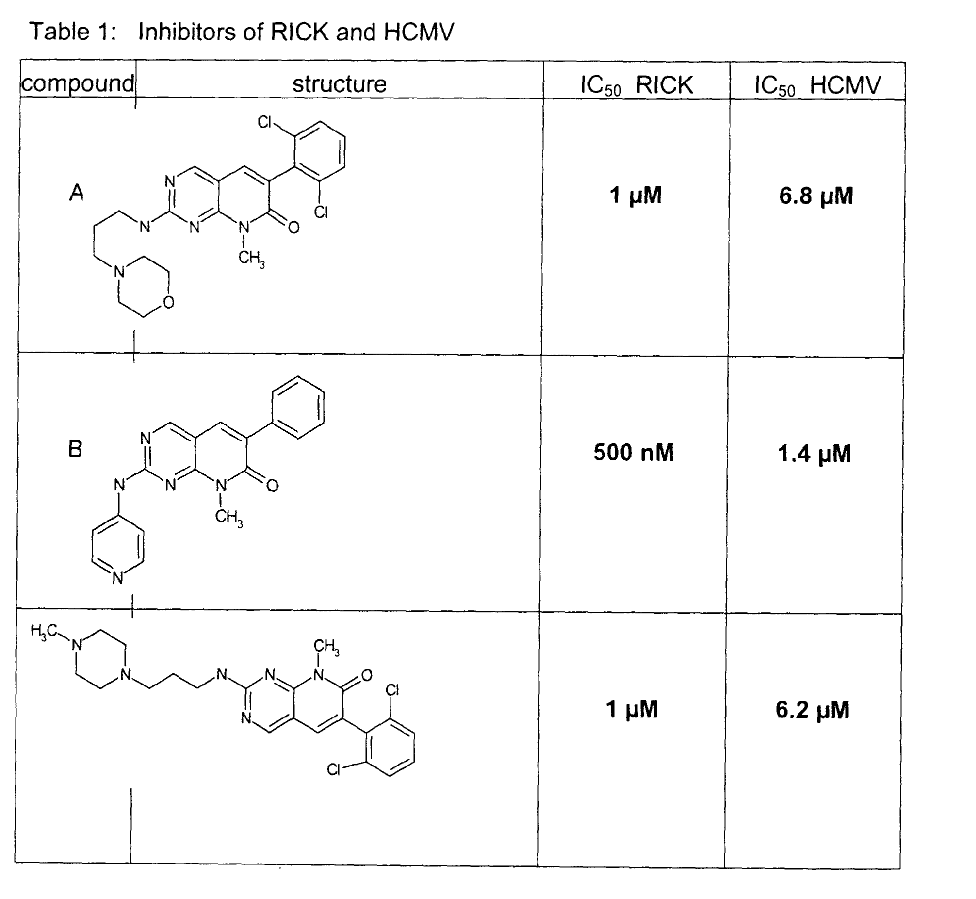

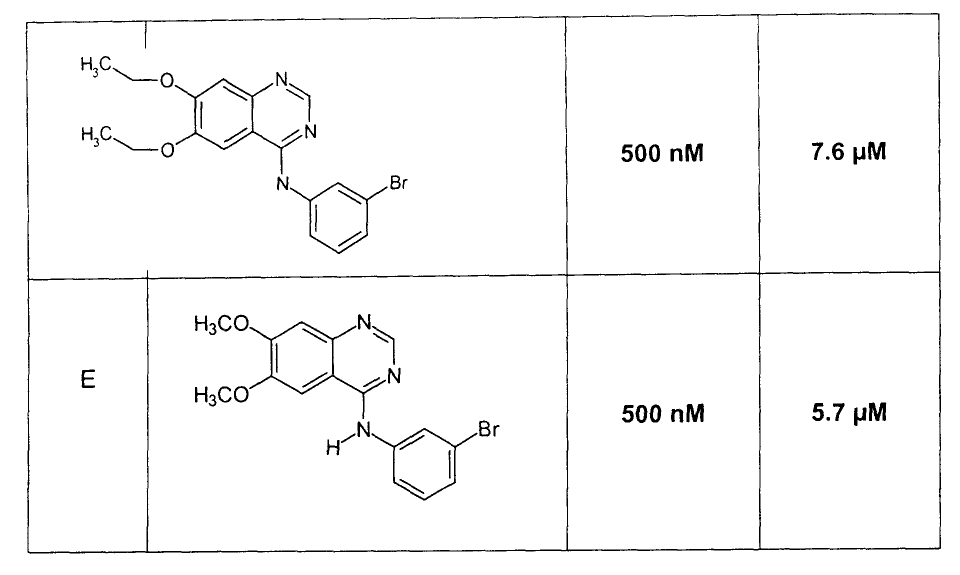

The compounds A to E have the following names:
- Compound A:: 6-(2,6-Dichlorophenyl)-8-methyl-2-(3-morpholin-4-yl-propylamino)-8*H*-pyrido[2,3-*d*]pyrimidin-7-one;
- Compound B:: 8-methyl-6-phenyl-2-(pyridin-4-yl-amino)-8*H*-pyrido[2,3-*d*]pyrimidin-7-one;
- Compound C:: 6-(2,6-Dichlorophenyl)-8-methyl-2-[3-(4-methylpiperazin-1-yl)-propylamino]-8*H*-pyrido[2,3-*d*]pyrimidin-7-one;
- Compound D:: (3-Bromophenyl)-(6,7-diethoxyquinazolin-4-yl)-amine;
- Compound E:: (3-Bromophenyl)-(6,7-dimethoxyquinazolin-4-yl)-amine.

From the results observed with the compounds shown in Table 1 it is proved that RICK is an important target for the treatment of HCMV and diseases associated with HCMV infection. Inhibitors of the human cellular protein kinase RICK may serve as new pharmaceutical substances for prophylaxis and/or treatment of Cytomegalovirus infection and/or diseases associated with CMV infection.

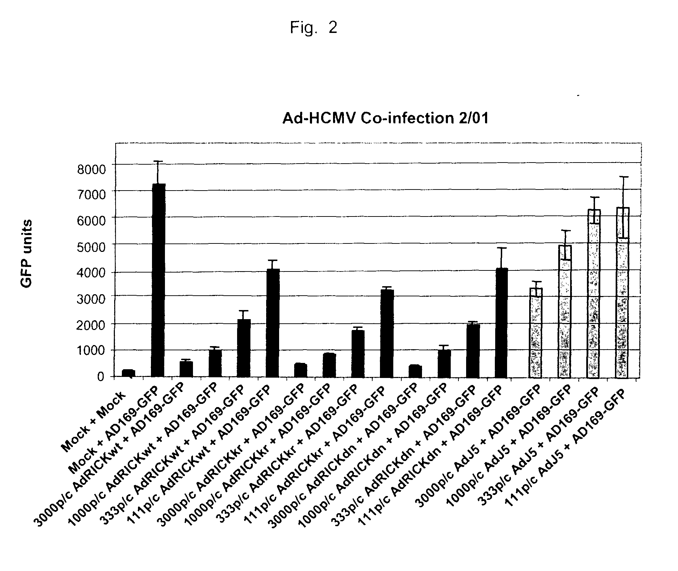

In addition to the chemical validation of RICK described above, a genetic validation of RICK in HCMV infection was performed. Wildtype and mutated RICK was expressed in HFF cells with a modified Adenovirus as vehicle (Example 9). The expression of both, wildtype and mutated RICK, caused a dramatic reduction in HCMV replication (cf. Fig. 2). Also these data confirm RICK as a valuable therapeutic target in HCMV treatment. As known in the art and as used herein, "RICK" refers to a protein kinase also known as "CARDIAK" and as "RIP2" which is a RIP-like kinase. RICK is essentially characterized as comprising an N-terminal serine-threonine kinase catalytic domain and a C-terminal region containing a caspase-recruitment domain (referred to as "CARD").

To perform initial tests for compounds that inhibit RIP activity in a cellular assay, RIP was transiently overexpressed in HEK-293 cells, immunoprecipitated and incubated with different concentrations of test compounds before in-vitro kinase assays were performed (Example 11). In order to identify compounds suitable for inhibiting the cellular kinase RIP and, thus, suitable for treating and/or preventing Cytomegalovirus infection and/or diseases associated with Cytomegalovirus infection the inventive method according to claim 1 was used. A test compound was contacted with the cellular kinase RIP according to the RIP assay protocoll disclosed in example 11. The test compound dissolved in DMSO is added to the RIP assay solution at concentrations between 100 nM and 50 µM. Radioactively labeled ATP was used as co-substrate of RIP and auto-phosphorylation was detected via measurement of incorporation of radioactivity into the RIP protein. Thereafter, phosphorylation rates with and without test compounds were compared. The three compounds listed in the following Table 2 showed inhibition of RIP kinase activity with an IC₅₀ between about 5 µM and 10 µM and an inhibition of HCMV with an IC₅₀ between about 12 µM and 15 µM, respectively. The IC₅₀ values of HCMV inhibition were obtained by the use of at least one assay protocol selected from a) virus replication assay, b) plaque assay, c) GFP infection assay, and d) indirect immunofluorescence analysis as disclosed in example 12.

Thus, the three compounds F to H mentioned below and/or pharmaceutically acceptable salts thereof can be used as inhibitors of the cellular protein kinase RIP and as pharmaceutically active compounds for the treatment and/or prophylaxis of HCMV infection. Furthermore, these compounds are suitable for the manufacture of a pharmaceutical composition for prophylaxis and/or treatment of Cytomegalovirus infection and/or diseases associated therewith.

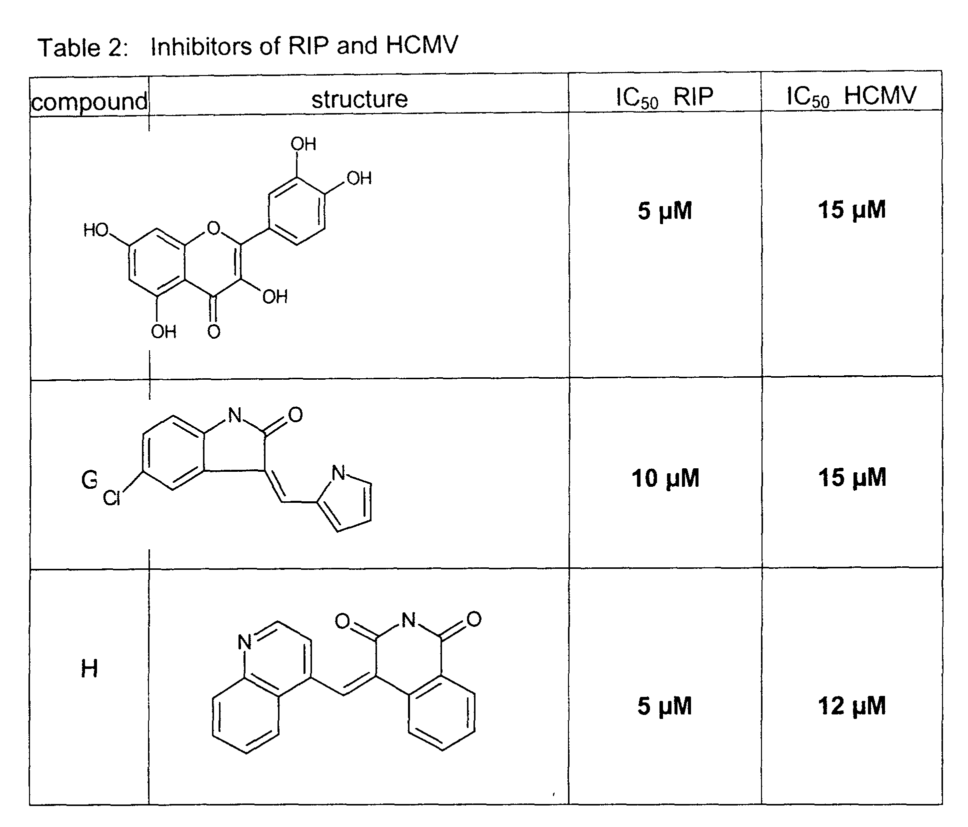

The compounds F to H have the following names:
- Compound F:: 2-(3,4-Dihydroxyphenyl)-3,5,7-trihydroxychromen-4-one;
- Compound G:: 5-Cloro-3-(1*H*-pyrrol-2-ylmethylene)-1,3-dihydroindol-2-one;
- Compound H:: 4-Quinolin-4-ylmethylene-4*H*-isoquinoline-1,3-dione.

From the results observed with the compounds shown in Table 2 it is proved that RIP is an important target for the treatment of HCMV and diseases associated with HCMV infection. Inhibitors of the human cellular protein kinase RIP may serve as new pharmaceutical substances for prophylaxis and/or treatment of Cytomegalovirus infection and/or diseases associated with CMV infection.

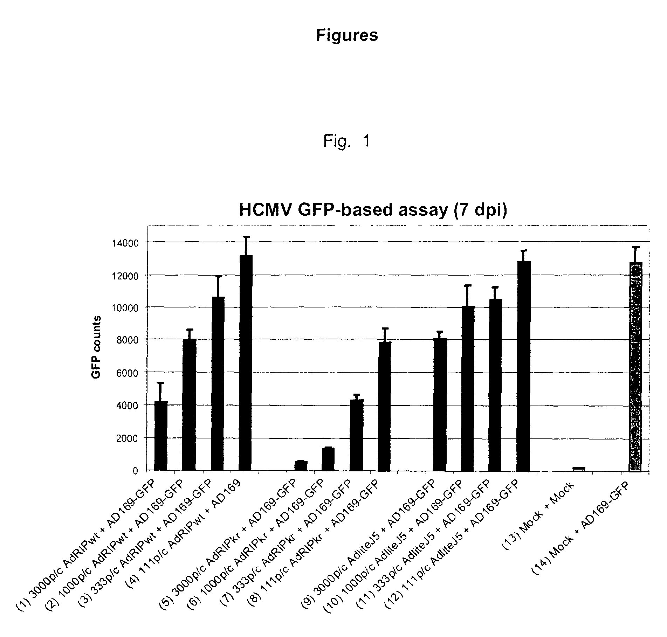

In addition to the chemical validation of RIP described above, a genetic validation of RIP in HCMV infection was performed. Wildtype and mutated RIP was expressed in HFF cells with a modified Adenovirus as vehicle (Example 9). The expression of mutated RICK, but not wildtype RIP, caused a dramatic reduction in HCMV replication (cf. Fig. 1). These data also confirm RIP as therapeutic target in HCMV treatment.

As known in the art and as used herein, "NIK" (Nck-Interacting Kinase; also known as "HGK" or "MAP4K4") refers to an NF-kappaB inducing serine/threonine kinase that interacts with the SH3 domains of Nck (an adaptor protein composed of one SH2 and three SH3 domains, known as a common target for a variety of cell surface receptors). NIK is most homologous to the Sterile 20 (Ste20) family of protein kinases, particularly GCK and MSST1 in that they bind neither Cdc42 nor Rac and contain an N-terminal kinase domain with a putative C-terminal regulatory domain. NIK is reported to promote neurite process formation and mediated anti-apoptotic signaling. NIK expression leads to IKK activation and induced nuclear translocation of NF-kappaB. NIK activates MEK1 phosphorylation and induces the Erk1/Erk2 MAPK pathway. NIK has been shown to be a MEK1-dependent activator of the MAPK pathway (Foehr et al., 2000. J. Biol. Chem. 275, 34021-34024). Overexpression of NIK has been reported to specifically activate the stress-activated protein kinase (SAPK) pathway; possibly upstream of MEKK1, a dominant-negative MEK kinase 1 capable of blocking NIK activation of SAPK (Su et al., 1997. EMBO 16(6):1279-90).

As known in the art and as used herein, "MKK3" (MAP kinase kinase 3; also known as "MEK3") refers to a protein kinase known to function in TNF-induced cytokine expression, and specifically phosphorylate and activate p38 MAP kinase (Blank et al., 1996. J. Biol. Chem. 271:5361-5368; Raingeaud et al., 1996. Mol. Cell. Biol. 16(3):1247-55). MKK3 gene disruption has been shown to cause a selective defect in the response of fibroblasts to the proinflammatory cytokine tumor necrosis factor, including reduced p38 MAP kinase activation and cytokine expression; suggesting that the MKK3 protein kinase is a critical component of a tumor necrosis factor-stimulated signaling pathway that causes increased expression of inflammatory cytokines (Wysk et al., 1999. PNAS USA 96(7):3763-8).

As known in the art and as used herein, "SRPK-2"( SR-protein-specific kinase 2) refers to a kinase known to phosphorylate SF2/ASF and believed to regulate the disassembly of the SR family of splicing factors in a tissue-specific manner (e.g., in testis, lung, and brain; Kuroyanagi et al., 1998. Biochem. Biophys. Res. Commun. 242(2):357-64). SRPK-2 is believed to function in spliceosome assembly and in mediating the trafficking of splicing factors (Wang et al., 1998. J. Cell. Biol. 140(4):737-50; Wang et al., 1999. Genomics 57(2):310-5).

In one embodiment, the present invention is directed to a method for treating CMV infection by administering a pharmaceutically effective amount of an inhibitor of one or more of the cellular kinases; RICK, RIP, NIK, MKK3, and/or SRPK-2.

As used herein, a cellular kinase "inhibitor" refers to any compound capable of downregulating, decreasing, suppressing or otherwise regulating the amount and/or activity of a cellular kinase. Inhibition of these cellular kinases can be achieved by any of a variety of mechanisms known in the art, including, but not limited to binding directly to the cellular kinase polypeptide (e.g., a RICK-inhibitor compound binding complex, or substrate mimetic), denaturing or otherwise inactivating the cellular kinase, or inhibiting the expression of the gene (e.g., transcription to mRNA, translation to a nascent polypeptide, and/or final polypeptide modifications to a mature protein), which encodes the cellular kinase. Generally, cellular kinase inhibitors may be proteins, polypeptides, nucleic acids, small molecules, or other chemical moieties.

Yet another aspect of the present invention is directed to pharmaceutical compositions useful for the prophylaxis and/or treatment of an individual afflicted with Cytomegalovirus infection and/or associated diseases. Said pharmaceutical composition comprises at least one pharmaceutically active compound capable of regulating at least partially the activity or the expression of one human cellular protein kinase selected from the group comprising RICK, RIP, NIK, MKK3, and SRPK-2 and/or capable of regulating the replication of CMV.

As used herein the term "regulating" refers either to the ability of an inhibitor to downregulate, decrease, reduce, suppress, inactivate, or inhibit at least partially the activity of an enzyme, or the expression of an enzyme and the virus replication or to the ability of an activator to upregulate, increase, stimulate, or activate at least partially the activity of an enzyme or the expression of an enzyme.

Suitable examples for inhibitors which are the pharmaceutically active components within the therapeutic compositions are the compounds A to H mentioned in Table 1 and 2. The compounds 6-(2,6-Dichlorophenyl)-8-methyl-2-(3-morpholin-4-yl-propylamino)-8H-pyrido[2,3-d]pyrimidin-7-one; 8-methyl-6-phenyl-2-(pyridin-4-yl-amino)-8H-pyrido[2,3-d]pyrimidin-7-one; 6-(2,6-Dichlorophenyl)-8-methyl-2-[3-(4-methylpiperazin-1-yl)-propylamino]-8H-pyrido[2,3-d]pyrimidin-7-one; (3-Bromophenyl)-(6,7-dimethoxyquinazolin-4-yl)-amine; (3-Bromophenyl)-(6,7-diethoxyquinazolin-4-yl)-amine; 2-(3,4-Dihydroxyphenyl)-3,5,7-trihydroxychromen-4-one; 5-Cloro-3-(1H-pyrrol-2-ylmethylene)-1,3-dihydroindol-2-one; 4-Quinolin-4-ylmethylene-4H-isoquinoline-1,3-dione and/or pharmaceutically acceptable salts of these compounds are useful for prophylaxis and/or treatment of Cytomegalovirus infection and/or diseases associated with Cytomegalovirus infection.

CMV therapeutics may be administered to cells from an individual *in vitro,* or may involve *in vivo* administration to the individual. Routes of administration of pharmaceutical preparations to an individual may include inhalation, oral and parenteral, including dermal, intradermal, intragastral, intracutan, intravasal, intravenous, intramuscular, intraperitoneal, intranasal, intravaginal, intrabuccal, percutan, rectal, subcutaneous, sublingual, topical or transdermal application, but are not limited the these ways of administration. For instance, the preferred preparations are in administratable form which is suitable for oral application. These administratable forms, for example, include pills, tablets, film tablets, coated tablets, capsules, powders and deposits. Administration to an individual may be in a single dose or in repeated administrations, and may be in any of a variety of physiologically acceptable salt forms, and/or with an acceptable pharmaceutical carrier, binder, lubricant, excipient, diluents and/or adjuvant. Pharmaceutically acceptable salt forms and standard pharmaceutical formulation techniques are well known to persons skilled in the art (see, for example, Remington's Pharmaceutical Sciences, Mack Publishing Co.).

As used herein, a "pharmaceutically effective amount" of a cellular kinase inhibitor is an amount effective to achieve the desired physiological result, either in cells treated *in vitro* or in a subject treated *in vivo.* Specifically, a pharmaceutically effective amount is an amount sufficient to inhibit, for some period of time, one or more of the clinically defined pathological processes associated with the viral infection. The effective amount may vary depending on the specific kinase inhibitor selected, and is also dependent on a variety of factors and conditions related to the subject to be treated and the severity of the infection. For example, if the inhibitor is to be administered *in vivo,* factors such as the age, weight and health of the patient as well as dose response curves and toxicity data obtained in preclinical animal work would be among those considered. If the inhibitor is to be contacted with the cells *in vitro,* one would also design a variety of pre-clinical *in* *vitro* studies to assess such parameters as uptake, half-life, dose, toxicity, etc. The determination of a pharmaceutically effective amount for a given agent is well within the ability of those skilled in the art.

As a result of the discovery of the upregulation of certain cellular kinases as part of the infection process of CMV, a novel diagnostic assay useful for the detecting CMV infection of an individual (or cell) is identified. The diagnostic assay of the present invention involves providing a sample from an individual or providing cells and/or cell lysates, and detecting activity in the sample of one or more cellular kinases selected from the group consisting of RICK, RIP, NIK, MKK3, and SRPK-2. In one embodiment, deviations in the expression levels of one or more of the identified cellular kinases in a test sample compared to a known normal expression levels (e.g., determined from a sample from a healthy individual) will be diagnostic of CMV.

It is apparent to a practitioner in the art that a sample useful for detecting CMV infection, whether of a subject individual or an isolated cell, refers to any cellular extract (including whole cells) from a tissue or body fluid (in the case of an individual) or cellular lysate (in the case of an isolated cell), which contains cellular components representative of cellular activity of one or more of the above-mentioned cellular kinases.

It is also apparent to a person of ordinary skill in the art that detection includes any method known in the art useful to indicate the presence, absence, or amount of a detection target. Such methods may include, but are not limited to, any molecular or cellular techniques, used singularly or in combination, including, but not limited to: hybridization and/or binding techniques, including blotting techniques and immunoassays; labeling techniques (chemiluminescent, colorimetric, fluorescent, radioisotopic); spectroscopic techniques; separations technology, including precipitations, electrophoresis, chromatography, centrifugation, ultrafiltration, cell sorting; and enzymatic manipulations (e.g., digestion).

Because the present disclosure teaches for the first time the upregulation of a group of cellular kinases specifically involved in the viral infection of CMV, the present invention is also directed to an assay useful for detecting novel compounds useful for treating CMV infection.

Assays of the present invention identify compounds useful for treating CMV operate by screening a test compound, or library of test compounds, for its ability to inhibit any one or more of the group of cellular kinases identified herein as characteristically upregulated during CMV growth and replication inside a cell. A variety of assay protocols and detection techniques are well known in the art and easily adapted for this purpose by a skilled practitioner. Such assays include, but are not limited to, high throughput assays (e.g., microarray technology, phage display technology), and *in vitro* and *in vivo* cellular and tissue assays.

In a related aspect, it is also an object of the present invention, in view of the discovery of cellular kinases specifically involved in CMV growth in a cell, to provide an assay component specially useful for detecting CMV in an individual (or a cell). Preferably the assay component comprises oligonucleotides capable of detecting activity of one or more of the cellular kinases RICK, RIP, NIK, MKK3, and SRPK-2 in a sample (e.g., by hybridization to mRNA from the sample), immobilized on a solid support. Most preferably the solid support would contain oligonucleotides of sufficient quality and quantity to detect all of the above-mentioned cellular kinases (e.g., a nucleic acid microarray).

Similarly, it is part of the object of the invention to provide an assay component specially useful for screening compounds useful for treating CMV. One preferred assay component comprises oligonucleotides that encode one or more of the cellular kinases RICK, RIP, NIK, MKK3, and SRPK-2, immobilized on a solid support. In another embodiment, the assay component comprises peptide fragments of one or more of the above-identified cellular kinases immobilized on a solid support. Once again the most preferred solid support embodiment would contain polymers of sufficient quality and quantity to detect all of the above-mentioned cellular kinases (e.g., a nucleic acid or a peptide microarray). A variety of assay supports and construction of the same are well known in the art and easily adapted for this purpose by a skilled practitioner (see, for example: Marshall, 1999. "Do-it-yourself gene watching" Science 286:444-447 (including insets); and Service, 2000. "Protein arrays step out of DNA's shadow" Science 289:1673).

It is preferred that mRNA is assayed as an indication of expression. Methods for assaying for mRNA include, but are not limited to, Northern blots, slot blots, dot blots, and hybridization to an ordered array of oligonucleotides. Nucleic acid probes useful for assay of a sample are preferably of sufficient length to specifically hybridize only to appropriate, complementary transcripts. Typically the oligonucleotide probes will be at least 10, 12, 14, 16, 18, 20 or 25 nucleotides in length. In some cases longer probes of at least 30, 40, or 50 nucleotides will be desirable.

The cDNA oligonucleotides immobilized on said membrane filter which are used for detecting the up- or downregulation of the above-mentioned human cellular protein kinases by hybridization to the radioactively labeled cDNA probes have the nucleotide sequences listed in table 3.

**Table 3:**

| Nucleotide sequences of cDNA-arrays | |
|---|---|
| Human cellular kinase | Sequence of immobilized DNA on arrays (in relation to the respective Acc No) |
| RICK | 914 bp - 2501 bp (AF027706) |
| RIP | 1421 bp - 2617 bp (U50062) |
| NIK | 231 bp - 3077 bp (Y10256) |
| MKK3 | 341 bp - 2030 bp (NM_002756) |
| SRPK-2 | 1238 bp - 2790 bp (U88666 ) |

The nucleoside sequences of the genes coding for the human cellular protein kinases RICK, RIP, NIK, MKK3, and SRPK-2 listed in Table 3 together with the amino acid sequences of said enzymes can be obtained from NCBI (National Library of Medicine: PubMed; Web address: www.ncbi.nlm.nih.gov/entrez). Sequence protocols of the five cellular protein kinases are attached to this application as a part of the description.

The polypeptide product of gene expression may be assayed to determine the amount of expression as well. Methods for assaying for a protein include, but are not limited to, Western blot, immunprecipitation, radioimmunoassay and peptide immobilization in an ordered array. It is understood, however, that any method for specifically and quantitatively measuring a specific protein or mRNA product can be used.

A variety of supports upon which nucleic acids or peptides can be immobilized are known in the art, for example filters, or polyvinyl chloride dishes. Any solid surface to which oligonucleotides or peptides can be bound, either directly or indirectly, either covalently or non-covalently, can be used. A preferred solid support is a microarray membrane filter or a "biochip". These contain particular polymer probes in predetermined locations on the array. Each predetermined location may contain more than one molecule of the probe, but each molecule within the predetermined location has an identical sequence.

The present invention incorporates by reference in their entirety techniques well known in the field of molecular biology. These techniques include, but are not limited to, techniques described in the following publications:
Ausubel, F.M. et al. eds., Short Protocols In Molecular Biology (4th Ed. 1999) John Wiley & Sons, NY. (ISBN 0-471-32938-X).
Old, R.W. & S.B. Primrose, Principles of Gene Manipulation: An Introduction To Genetic Engineering (3d Ed. 1985) Blackwell Scientific Publications, Boston. Studies in Microbiology; V.2:409 pp. (ISBN 0-632-01318-4).
Miller, J.H. & M.P. Calos eds., Gene Transfer Vectors For Mammalian Cells (1987) Cold Spring Harbor Laboratory Press, NY. 169 pp. (ISBN 0-87969-198-0).
Mayer, R.J. & J.H. Walker eds., Immunochemical Methods In Cell and Molecular Biology (1987) Academic Press, London. 325 pp. (ISBN 0-12480-855-7).
Sambrook, J. et al. eds., Molecular Cloning: A Laboratory Manual (2d Ed. 1989) Cold Spring Harbor Laboratory Press, NY. Vols. 1-3. (ISBN 0-87969-309-6).
Winnacker, E.L. From Genes To Clones: Introduction To Gene Technology (1987) VCH Publishers, NY (translated by Horst Ibelgaufts). 634 pp. (ISBN 0-89573-614-4).

The present invention further incorporates by reference in their entirety techniques well known in the field of microarray construction and analysis. These techniques include, but are not limited to, techniques described in the following patents and patent applications describing arrays of biopolymeric compounds and methods for their fabrication: U.S. Pat. Nos. 5,242,974; 5,384,261; 5,405,783; 5,412,087; 5,424,186; 5,429,807; 5,436,327; 5,445,934; 5,472,672; 5,527,681; 5,529,756; 5,545,531; 5,554,501; 5,556,752; 5,561,071; 5,599,895; 5,624,711; 5,639,603; 5,658,734; 5,807,522; 6,087,102; WO 93/17126; WO 95/11995; WO 95/35505; EP 742 287; and EP 799 897. Techniques also include, but are not limited to, techniques described in the following patents and patent application describing methods of using arrays in various applications: U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,994,076; 6,033,860; 6,040,138; 6,040,140; WO 95/21265; WO 96/31622; WO 97/10365; WO 97/27317; EP 373 203; and EP 785 280.

It is readily apparent to those skilled in the art that other suitable modifications and adaptations of the compositions and methods of the invention described herein are obvious and may be made without departing from the scope of the invention or the embodiments disclosed herein. Having now described the present invention in detail, the same will be more clearly understood by reference to the following examples, which are included for purposes of illustration only and are not intended to be limiting of the invention.

### Examples

### Materials and Methods

### EXAMPLE 1:

### Signal Transduction cDNA Microarray Construction

To study the cellular pathology associated HCMV, a unique microarray of more than 1100 signal transduction cDNAs was created.

In order to manufacture cDNA-arrays on membranes, the following strategy was employed: cDNAs encoding parts of or full length proteins of interest (referred to as "target cDNAs") were cloned into the plasmid BLUESCRIPT II KS⁺ (Stratagene, USA). Large scale purifications of these plasmids were performed according to standard techniques, and 200µl aliquots (1µg/µl plasmid concentration) were transferred into appropriate 96 well plates. The plates were then sealed with sealing tape, incubated for 10 minutes at 95°C, and chilled on ice for 5 minutes. 10µl of 0.6N NaOH were added, and the mix was then stored for 20 minutes at room temperature. Following the incubation at room temperature, 10µl 2.5M Tris-HCI (Tris-(hydroxymethyl)-aminomethane-hydrochloride) pH7.1 and 20µl 40x SSC (6M Sodium chloride - 0.6M tri-Sodium citrate buffer) was added.

Target cDNAs were spotted onto Nylon or Nitrocellulose membranes using a BIOGRID (BioRobotics, UK) equipped with a 0.7mm pintool. 200-350ng of plasmid-encoding target cDNAs were transferred onto the membranes and crosslinked to the membranes using ultraviolet light (1.2x10⁵ µJ/cm²) treatment. The arrays were stored for use in subsequent experiments (described below) at room temperature.

### EXAMPLE 2:

### HCMV Infection

To examine the effects of HCMV infection on cellular signal transduction activity, HCMV-infected cells were generated for comparison to control (i.e., uninfected) cells.

Primary human foreskin fibroblasts (HFF) were grown close to confluency in MEM medium (Minimum Essential Medium, Life Technologies) supplemented with 20% fetal calf serum at 37°C and 5% CO₂ to obtain ∼6x10⁶ cells per tissue culture flask. Virus adsorption to the cells was performed with the HCMV strain AD169 at different (0.2, 1, and 3) multiplicities of infection (MOI) for 90 minutes in a volume of 5ml at 37°C. The viral inoculum was removed, and cells were cultured in 50ml of MEM medium supplemented with 20% fetal calf serum and 150µg/ml cycloheximid at 37°C and 5%CO₂ for 7, 24, 48, or 72 hours, respectively.

### EXAMPLE 3:

### Isolation and Purification of Poly A⁺ RNA

In order to perform differential expression analysis using the cDNA microarray described in Example 1, RNA extraction and purification on the HCMV-infected and uninfected cells was performed using techniques known in the art.

Briefly, after incubation (for the respective time-intervals) of infected and control cells, cells were washed twice with phosphate buffered saline (PBS) and then trypsinized. Cells were removed from the culture dish by resuspension with PBS. Cells were then sedimented, and directly lysed by repetitive pipetting in 1ml of Tri reagent (Molecular Research Centre, Inc., USA) per 1×10⁶ cells.

Cell lysates were stored at room temperature for 5 minutes, and then centrifuged (12,000xg) for 15 minutes at 4°C. The supernatant was mixed with 0.1ml of 1-bromo-3-chloropropane per 1ml of Tri reagent and shaken vigorously. The resultant suspension was stored for 5 minutes at room temperature, and then centrifuged (12,000xg) for 15 minutes at 4°C.

Following centrifugation, the colorless upper phase was transferred into new tubes, mixed with 5µl of poly-acryl-carrier (Molecular Research Centre, Inc., USA), and vigorously shaken with 0.5ml of isopropanol per 1ml of Tri reagent. The samples were stored at room temperature for 5 minutes and then centrifuged (12,000xg) for 8 minutes at 4°C. The supernatant was removed and the RNA pellet washed twice with 1ml of 75% ethanol. The pellet was dried and resuspended in RNase-free buffer at a concentration of 1µg RNA per 1µl buffer.

Purification of poly A⁺ RNA from total RNA was performed using the OLIGOTEX system (Qiagen, Germany) following manufacturer's instructions. In brief, 100-200µg of total RNA was brought up to 250µl with RNase-free water, and 250µl of buffer OBB (20mM Tris/HCl pH7.5, 1M NaCI, 2mM EDTA, 0.2% SDS) and 15µl of OLIGOTEX suspension was added. The samples were incubated for 3 minutes at 70°C, and placed at room temperature for 10 minutes. The samples were centrifuged for 2 minutes (12,000xg), and the supernatant removed. The remaining pellet was resuspended in 400µl buffer OW2 (10mM Tris/HCl pH7.5, 150mM NaCI, 1mM EDTA). The suspension was transferred to a spin column (supplied with the system) and centrifuged at 12000xg for 1 minute at room temperature. The spin column was transferred to a new tube and 400 µl of buffer OW2 was applied on the column. The spin column was centrifuged (12,000xg) for 1 minute at room temperature. The spin column was transferred to a new tube and the RNA eluted from the column by the addition of 50µl buffer OEB (5mM Tris/HCl pH7.5) (at 70°C) to the column, resuspension of the Oligotex-resin, and centrifugation (12,000xg) for 1 minute at room temperature.

Any genomic DNA contamination of the RNA preparations was eliminated by enzymatic digestion using DNase I. 6µl of 10x DNase buffer (Promega, USA) and 4µl of RQ-DNase (Promega, USA) were added to 50µl of the RNA-buffer solution, and the reaction mixture was incubated for 15 minutes at 37°C. Stop-buffer (6µl; Promega, USA) was then added, the mixture brought to 200 µl final volume with TE buffer (10mM Tris/HCl, 1mM EDTA), and Phenol/Chloroform extractions were performed twice. The RNA-containing phase was transferred to new reaction tubes and RNA was precipitated using 5M NaCI (final concentration of 0.2M), 1µl poly-acryl-carrier (Molecular Research Centre, Inc., USA) and 500 µl of 100% ethanol. The solution was centrifuged for 10 minutes at 4°C, the RNA pellet washed with 1ml of 80% ethanol, dried, and resuspended in 30µl TE buffer. Poly A⁺ RNA suspension samples were stored at -70°C for use in subsequent experiments.

### EXAMPLE 4:

### Preparation of Radioactively Labeled cDNA Probes from RNA

To prepare test and control samples for microarray analysis, RNA samples isolated and purified from HCMV-infected and control cells (prepared as described in Example 3) were used to generate radioactively labeled cDNA probe. Many techniques to generate labeled cDNA constructs from cellular RNA extracts are known in the art and applicable to the present invention. Two of those protocols were used in this example to generate radiolabeled cDNA from RNA samples: the first technique involved reverse transcribing cDNA from the RNA sample in the presence of radioactively labelled dATP; the second technique involved first strand cDNA synthesis from the RNA sample, followed by random priming with radioactively labelled dATP.

For reverse transcription of cDNA from the RNA sample in the presence of radioactively labelled dATP, 1µg of primer TXN (5'-TTT TTT TTT TTT TTT TXN-3'; SEQ ID NO:1; with T = dTTP; N = dATP, dCTP, dGTP or dTTP; X = dATP, dCTP or dGTP) and total RNA (1 to 15 µg) or poly A⁺ RNA (20 to 500 ng) were combined in 12 µl bidistilled DEPC-treated H₂O (DEPC: diethylpyrocarbonate) and shaken for 5-15 minutes at 60°C. The mixture was then incubated at 4°C for 2-10 minutes, and centrifuged (10,000xg) for 30 seconds.

After centrifugation, 7µl of a labelling mix (100µCi γ[³³P]-ATP (Amersham, UK); vacuum dried and resuspended in 4µl first strand buffer (Life Technologies, USA); 2µl 0.1M DTT (dithiothreitol); and 1µl labelling solution -- 4mM dCTP, dGTP, dTTP each and 80 µM dATP final concentration) was added to the RNA solution. 1µl SUPERSCRIPT II reverse transcriptase (Life Technologies, USA) was added and the reaction incubated for 10 minutes at room temperature and then for 60 minutes at 38°C. Following the reaction incubation, 5µl 0.5M EDTA (ethylene diamine tetraacetate) and 25 µl 0.6M NaOH was added to the reaction mixture and shaken vigorously for 30 minutes at 68°C.

Unincorporated nucleotides were removed from the labelling reaction using PROBEQUANT G-50 columns (Amersham, UK). The column (with bottom closure and lid removed) was shaken vigorously and centrifuged (735xg) for 1 minute in an appropriate reaction tube. The column was placed into a new reaction tube, the probe was applied onto the center of the column material and the column was centrifuged (735xg) for 2 minutes. The flow-through was transferred into new reaction tubes and bidistilled H₂O added to 100 µl final volume. 5M NaCI, 1µl poly-acryl-carrier (Molecular Research Centre, Inc., USA) and 250 µl ethanol was added, and the probe precipitated by centrifugation (12,000xg) for 15 minutes. The supernatant was discarded and the pellet dried for subsequent use.

For the alternate labelling technique (random priming with radioactively labelled dATP after first strand cDNA synthesis), the following procedure was followed: 1µg primer TXN (see above) was added to 20-500ng of poly A⁺ RNA in 12 µl final volume, incubated for 5 minutes at 60°C, followed by an addition incubation for 2-10 minutes on ice. The mix was centrifuged (12,000xg) for 30 seconds, and 4µl of first strand buffer (Life Technologies, USA), 2µl 0.1M DTT, 1µl 10mM dNTP and 1µl SUPERSCRIPT II reverse transcriptase (Life Technologies, USA) was added. The reaction was incubated for 10 minutes at room temperature, followed by an additional incubation for 60 minutes at 38°C. Following the reaction incubation, 5µl 0.5M EDTA and 25 µl 0.6M NaOH was added to the reaction mixture and shaken vigorously for 30 minutes at 68°C.

Unincorporated nucleotides were removed as described above; however, the final pellet was resuspended in 30µl bidistilled H₂O.

15 µl of the resuspended cDNA solution was transferred to new reaction tubes, incubated for 5 minutes at 95°C, chilled on ice for 5 minutes, and centrifuged for 30 seconds. Following manufacturer's instructions accompanying the Random Primers DNA Labelling system (Life technologies, USA), 15 µl buffers mixture, 2µl of each dCTP, dGTP and dTTP (provided with the system) were added to the cDNA. 5µl γ[³³P]-ATP (Amersham, UK) was added and the mixture adjusted to 49µl final volume with bidistilled H₂O. The reaction was started by addition of 1µl Klenow enzyme (supplied with the system), and incubated for 60 minutes at 25°C 5µl. Stop solution (provided with the system) was added and unincorporated nucleotides were removed by column purification as described above.

### EXAMPLE 5:

### Hybridization of Labeled cDNA Probe to cDNA Array

To screen HCMV-infected cells compared to uninfected cells for differential activation of cellular signal transduction, labeled cDNA probes (generated according to Example 4) were exposed to a signal transduction cDNA microarray (generated as described in Example 1) following hybridization techniques known in the art.

Sample pellets from Example 4 were resuspended in 10µl C₀T DNA (1µg/µl, Roche Diagnostics, Germany), 10µl yeast tRNA (1µg/µl Sigma, USA) and 10µl poly A (1µg/µl, Roche Diagnostics, Germany). Herring sperm DNA (to a final concentration of 100µg/ml), 5µl 10% SDS (Sodiumdodecylsulfate), and 25 µl 20x SSPE was added, and adjusted 100µl final volume with bidistilled H₂O. The mix was incubated for 5 minutes at 95°C, centrifuged (10,000xg) for 30 seconds, and vigorously shaken for 60 minutes at 68°C. A 1µl aliquot of the probe was used to measure the incorporation of radioactive dATP with a scintillation counter. Probes with at least a total of 20x10⁶ cpm were used for the screen assay.

Arrays were prehybridized in hybridization solution for at least 30 minutes in a roller bottle oven at 42°C. Following prehybridization, radiolabelled probe was added to the hybridization solution and hybridization was continued for 20-40 hours.

Following hybridization, the probe was discarded and the array subjected to a series of washes. Initially the arrays were washed twice in wash solution A (2xSSC) in the roller oven at room temperature. Wash solution A was then replaced with wash solution B (2x SSC, 0.5% SDS), preheated to 60°C, and arrays were washed twice for 30 minutes at 60°C. Wash solution B was then replaced with wash solution C (0.5x SSC, 0.5% SDS), preheated to 60°C, and arrays were washed twice for 30 minutes at 60°C.

The moist arrays were wrapped in airtight bags and exposed for 8-72 hours on erased phosphoimager screens (Fujifilm, Japan).

### EXAMPLE 6:

### Signal Transduction cDNA Array Analysis

To demonstrate differential activation of cellular signal transduction in HCMV-infected cells compared to uninfected cells, hybridized cDNA arrays from infected and uninfected samples were analyzed.

Exposed phosphoimager screens (from Example 5) were scanned with a resolution of 100µ and 16bits per pixel using a BAS-1800 (Fujifilm, Japan). The data were imported into the computer program, ARRAYVISION (Imaging Research, Canada), and analyzed according the computer program's specification. Hybridization signal strength is indicative of the quantity of RNA molecules present in the probe. Differentially expressed genes were identified according to the ratio of signal strength after normalization to the overall intensity of the arrays.

Signal transduction cDNA microarray analysis of radiolabelled cDNA-probes from HCMV-infected (strain AD169) versus non-infected primary human foreskin fibroblasts to cDNA-arrays revealed significant upregulation of the cellular kinase cDNAs:
RICK (2-fold at 3 hours post infection; 3.6-fold at 7 hours post infection);
RIP (2.6-fold at 3 hour post infection; 2.2-fold at 24 hour post infection);
NIK (4-fold at 7 hour post infection);
MKK3 (2-fold at 3 hour post infection; 2.5-fold at 7 hour post infection); and
SRPK-2 (2.2-fold at 7 hour post infection)
compared to uninfected human foreskin fibroblasts cells.

### EXAMPLE 7:

### Northern Blot Analysis

To confirm the results of the microarray analysis of Example 6, northern blot analysis was performed according to techniques well known in the art.

HCMV-infected and uninfected cells (from Example 2) cells were pelleted and the total RNA was prepared as follows: Following centrifugation and removal of the supernatant, cells were lysed in 1ml of Trizol reagent (ready-to-use-reagent from Gibco-BRL) per 1.5 10⁶ cells. The Tri reagent/cell lysate was transferred to an eppendorff tube and centrifuged (13,000rpm) for 15 minutes at 4°C. The supernatant was transferred to a new eppendorff tube and 0.1ml of BCP (1-bromo-3-chlorpropane) for each ml of Tri reagent was added. Samples were vortexed for 15 seconds, incubated for 5 min at room temperature, and then centrifuged (13,000rpm) for 15 minutes at 4°C. The upper aqueous phase was transferred to a new eppendorff tube, 0.5 ml isopropanol was added for each ml of Tri reagent (Molecular Research Center, Inc., USA), vortexed, and incubated for additional 8 min at room temperature, and centrifuged (13,000rpm) for 10 min at 4°C. The supernatant was aspirated, and the precipitated RNA was washed twice with ice-cold 75% ethanol and air-dried. The RNA pellet was resuspended in 50µl Tris-HCl pH 7.5.

The quantity of the RNA for each sample was determined by UV-spectroscopy, and the quality was determined via gel electrophoresis on a formaldehyde-containing 1.2% agarose gel.

RNA samples of 10µg each were size-fractionated by 1.2% formaldehyde agarose gel electrophoresis and transferred to synthetic membrane filters (Hybond N, Amersham) with 20XSSC (1 X SSC is 150 mM NaCI, 15 mM C₆H₅Na₃O₇ x 2H₂O, pH 7.0) overnight. RNA was immobilized to the filter using UV-light for crosslinking (120 mJ/cm² for 25 seconds).

Membrane filters were firstly prehybridized for 4 hours at 65°C in a prehybridization solution containing 5 X SSC, 10 X Denhardt's solution (1 X Denhardt's solution is 0.02% bovine serum albumine, 0.02% polyvinyl pyrrolidone, 0.02% ficoll), 20mM sodium phosphate, pH 7.0, 7% SDS, 100µg/ml sonicated salmon sperm DNA, and 100µg/ml. Hybridization was performed at 65°C in the prehybridization buffer containing 10% dextran sulphate, plus added radiolabelled probe for 16 hours.

Membrane filters were hybridized to oligonucleotide probes specific for a particular cellular kinase identified in Example 6. Probes sequences included the following:

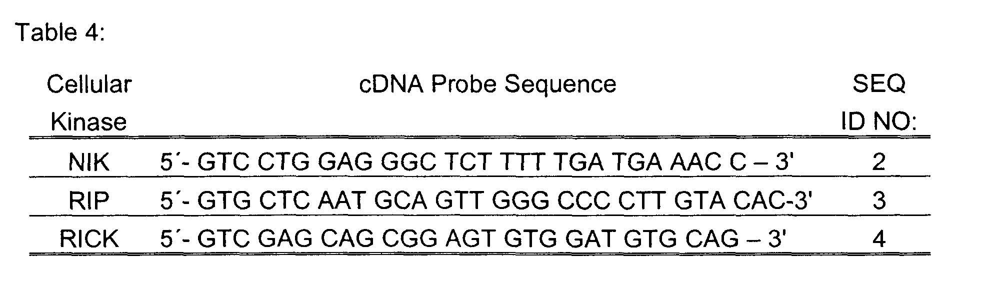

The oligonucleotides were radiolabelled at their 3' ends with (alpha-32P) deoxyadenosine 5'- triphosphate (³²P-α-dATP) (Amersham) employing the Terminal Transferase kit (Roche) following manufacturer's instructions.

Unincorporated ³²P-α-dATP nucleotides were removed similar to the protocol described in Example 4: After vortexing the PROBEQUANT Sephadex G-50 (Amersham, UK) column (with bottom closure and lid removed), the column was placed in a 2 ml tube and centrifuged for 1 minute at 735xg. The column was placed in a new 1.5ml eppendorff tube (without a cap), and the radioactive probe was pipetted carefully on the center of the preformed resin. Centrifugation (735xg) for 2 minutes effectively removed the unincorporated ³²P-α-dATP nucleotides.

Hybridized filters were washed once in 5% SDS, 3 X SSC, 10 X Denhardt's solution, 20mM sodium phosphate, pH 7.0 for 30 min at 65°C. A second wash step followed in 1 X SSC, 1% SDS at 65°C for 30 min.

Filters were exposed at -80°C to Kodak XAR-5 films using intensifying screens.

Northern blot analysis confirmed upregulation of cellular kinase mRNA: RICK, RIP, and NIK in HCMV-infected cells compared to uninfected cells, consistent with results obtained from microarray analysis.

### EXAMPLE 8:

### Western Blot Analysis

To further confirm the results of the microarray analysis of Example 6 and northern blot analysis of Example 7, western blot analysis was performed according to techniques well known in the art.

HCMV-infected and uninfected cells (from Example 2) were pelleted and polypeptide extracts prepared as follows: Infected and uninfected cell samples (from various time intervals) were lysed with 420 µl of lysis buffer (20mM Hepes (N-[2-hydroxyethyl]piperazine-N'-[2-ethansulfonic acid]) pH7.5, 150 mM NaCI, 1% TRITON X-100 (t-octylphenoxypolyethoxyethanol), 10% glycerol, 1mM PMSF (phenylmethylsulfonyl fluoride), 10µg/ml Aprotinin, 1mM ortho-vanadat) on ice. Lysed cells were cleared from debris by centrifugation (15 minutes, 13000 rpm, 4°C), dissolved in 1x Laemmli buffer, denatured for 5 minutes at 100°C and submitted to SDS-PAGE (gradient gel 7% - 12%).

Gels were blotted onto nitrocellulose filters (Amersham, UK) for 3 hours (0.8mA/cm2). Detection of expression of the identified host cell kinases was performed using the following target specific antibodies: OPA1-01023 polyclonal rabbit anti-RICK antibody (Dianova); H-207 polyclonal rabbit anti-RIP antibody (Santa Cruz Biotechnology); I-20 polyclonal rabbit anti-MKK3 antibody (Santa Cruz Biotechnology); S80620 murine anti-SRPK2 antibody (Transduction Laboratories); anti-NIK rabbit serum (generated by SIGMA Genosys Biotechnologies using the NIK-peptide 5'-CNPTNTRPQSDTPEIRKYKKRFN-3', SEQ ID NO:5, for immunization). All antibodies were used according to the manufacturer's instructions. Detections were performed with the ECL Kit (Amersham, UK).

Western blot analysis confirmed the transcriptional upregulation of infected host cell kinase mRNAs resulted in increased expression of the respective proteins:
A single ∼60kDa band representing RICK was upregulated between 7-24 hours post HCMV infection;
A single ∼74kDa band representing RIP was upregulated between 7-72 hours post HCMV infection;
A single ∼135kDa band representing NIK was upregulated between 24-72 hours post HCMV infection;
A single ∼35kDa band representing MKK3 was upregulated between 7-72 hours post HCMV infection; and
A single ∼115kDa band representing SRPK2 was upregulated between 24-72 hours post HCMV infection.

### EXAMPLE 9:

### Genetic Validation

HFF cells were infected with Adenovirus expressing various kinase constructs at different particles per cell ratios (p/c). The adenovirus used here were all E1, E3 defective derivatives of adenovirus type 5 (reviewed in Russell WC (2000) Update on adenovirus and its vectors. J Gen Virol. 81:2573-604). Briefly, the cDNA of interest was cloned into a transfer plasmid bearing the CMV IE promoter enhancer (IE: immediate early) and the rabbit beta-globin intron/polyadenylation signal. This expression cassette was inserted into a bacterial plasmid borne-adenovirus genome using recombination in bacteria (Chartier C., E. Degryse, M. Gantzer, A. Dieterle, A. Pavirani, and M. Mehtali. 1996. Efficient generation of recombinant adenovirus vectors by homologous recombination in *Escherichia coli.* J. Virol. 70:4805-4810.). Virus was amplified in HEK 293 cells and purified from cell lysates using CsCI density gradient centrifugation as described (Cotten, M., Baker A., Birnstiel M.L., Zatloukal, K., Wagner, E. (1996) Adenovirus polylysine DNA conjugates. in Current Protocols in Human Genetics, Eds. N. C. Dracopoli, J. L. Haines, B.R. Korf, D.T. Moir, C.C. Morton, C.E. Seidman, J.G. Seidman, D.R. Smith; John Wiley and Sons, Inc. New York. pp. 12.3.1-12.3.33.). The control viruses AdJ5 was previously described (Glotzer J.B., Saltik M., Chiocca S., Michou A.I., Moseley P. and Cotten M. (2000) Activation of heat-shock response by an adenovirus is essential for virus replication. Nature 407:207-11).

Two days after plating HFF cells, cultures were infected with CMV strain Ad169-GFP. Replication of CMV was estimated after one week (7 dpi) utilizing the GFP-signal expressed as GFP counts.

Fig. 1 shows the reduction rates in HCMV replication of HHF cells pre-infected with Adeno virus containing the RIP wildtype sequence (AdRIPwt; 1 - 4) and a RIP inactive mutant (AdRIPkr; 5 - 8).

No HCMV-infection resulted in hardly any signal (mock, 13), while infection with HCMV yielded in about 13.000 GFP counts (AD169-GFP; 14). Pre-infection with increasing amounts of control Adeno virus (AdliteJ5) caused a slight reduction in HCMV replication (9 - 12). There was a clear difference, when HFF cells were pre-infected with Adeno virus containing the RIP wildtype sequence (AdRIPwt; 1 - 4) and a RIP inactive mutant (AdRIPkr; 5 - 8). The lysine (K) at amino acid position 45 is mutated to an arginine (R), which renders the kinase inactive. This mutation was introduced into the human RIP cDNA utilizing the QuikChange™ Site-directed Mutagenesis Kit (Stratagene, CA, USA) according to the instructions of the manufacturer. Expression of the mutated RIP kinase efficiently blocked HCMV replication (5 - 8), while the wildtype sequence was less potent in doing so (1 - 4).

Fig. 2 shows the reduction rates in HCMV replication of HHF cells pre-infected with Adeno virus containing the RICK wildtype sequence (AdRICKwt) and two RICK inactive mutants (AdRICKkr and AdRICKdn). In one construct (AdRICKkr), the lysine (K) at position 47 is mutated to an arginine (R). In the other construct (AdRICKdn), the aspartate at position 146 is mutated to an asparagine. Both changes in sequence render the kinase inactive. The mutations were introduced into the human RICK cDNA utilizing the QuikChange™ Site-directed Mutagenesis Kit (Stratagene, CA, USA) according to the instructions of the manufacturer.

Similar experiments as described for RIP (Example 9, Fig. 1) were also performed with RICK. No HCMV-infection resulted in hardly any signal (Mock + Mock), while infection with HCMV yielded in about 7.000 GFP counts (Mock + AD169-GFP). Pre-infection with increasing amounts of control Adeno virus (AdliteJ5, from 111 to 3000 particles per cell) caused a slight reduction in HCMV replication. There was a clear difference, when HFF cells were pre-infected with Adeno virus containing the RICK wildtype sequence (AdRICKwt and two RICK inactive mutants (AdRICKkr and AdRICKdn). All three RICK constructs efficiently reduced HCMV-replication.

### EXAMPLE 10:

### RICK-Kinase Assay

To obtain active RICK kinase the human RICK-cDNA was fused with a DNA sequence coding for the HA-tag and cloned into the eucaryotic expression vector pcDNA3 (Invitrogene). This construct was transfected into human embryonic kidney cells (HEK 293) via the calcium-phosphate co-precipitation method. One day after transfection medium was replaced by fetal calf serurum-free medium and two days after transfection cells were washed with PBS and harvested and lysed in RIPA-buffer (150 mM NaCI, 1 mM EDTA, 1% Tritron X-100, 1% Na-desoxycholate, 0.1 % SDS, 10 mM Tris-HCl pH 7.5). The RICK-HA fusion protein was immunoprecipitated from 250 µl cleared lysate (i.e. lysate of one well of a six-well plate) utilizing an anti-HA antibody from Roche Pharmaceuticals and Protein A sepharose. After addition of 500 µl of HNTG-buffer (50 mM HEPES pH 7.5, 150 mM NaCI, 1 mM EDTA, 10% glycerine, 0.1% Triton X-100) the sample was rotated for 3 hrs at 4°C. After washing the immunoprecipitate twice with 0.5 ml HNTG-buffer and twice with 0.5 ml assay-buffer (25 mM Tris-HCI pH 7.5, 3 mM MgCl₂, 0.5 mM MnCl₂, 0.5 mM DTT and 50 mM NaCI), the kinase reaction was performed directly on the beads in 40 µl assay buffer containing 2.5 µCi γ[³³P]-ATP and various concentrations (between 100 nM and 50 µM) of compounds of Table 1. After 30 min at 30°C, the reaction was stopped by addition of 40 µl 3X Laemmli-buffer (16% glycerol, 1.01M β-mercaptoethanol, 5% SDS, 200mM Tris/HCl pH 6.8, 8% bromphenolblue). Phosphorylation products were analyzed by SDS-PAGE and auroradiography (x-ray film and phosphor imager).

### EXAMPLE 11:

### RIP-Kinase Assay

To obtain active RIP kinase the human RIP-cDNA was fused with a DNA sequence coding for the HA-tag and cloned into the eucaryotic expression vector pcDNA3 (Invitrogene). This construct was transfected into human embryonic kidney cells (HEK 293) via the calcium-phosphate DNA co-precipitation method. Two days after transfection cells were washed with PBS and harvested and lysed in lysis-buffer (150 mM NaCI, 1 mM EDTA, 1% Tritron X-100, 20 mM Tris-HCI pH 7.5 and freshly added: 30 mM NaF, 10 µg/ml Aprotinine, 10 µg/ml Leupeptine, 2 mM Na-pyrophosphate). The RIP-HA fusion protein was immunoprecipitated from 250 µl cleared lysate (i.e. lysate of one well of a six-well plate) utilizing an anti-HA antibody from Roche Pharmaceuticals and Protein A sepharose. The sample was rotated for 3 hrs at 4°C. The immunoprecipitates were washed twice with 0.75 ml lysis-buffer, twice with 0.75 ml high salt-buffer (1 M NaCI, 1 mM EDTA, 1% Tritron X-100, 20 mM Tris-HCI pH 7.5 and freshly added: 30 mM NaF, 10 µg/ml Aprotinine, 10 µg/ml Leupeptine, 2 mM Na-pyrophosphate), twice with 0.75 ml lysis-buffer and twice 0.75 ml with kinase assay buffer (10 mM MgCl₂, 10 mM MnCl₂, 10 mM benzamidine, 0.5 mM EDTA). The kinase reaction was performed directly on the beads in 40 µl kinase assay buffer containing 2.5 µCi γ[³²P]-ATP and various concentrations (between 100 nM and 50 µM) of compounds of Table 2. After 30 min at 30°C, the reaction was stopped by addition of 40 µl 2X Laemmli-buffer. Phosphorylation products were analyzed by SDS-PAGE and autoradiography (x-ray film and phosphor imager).

### EXAMPLE 12:

### Virus Replication Assay

### Cell culture and virus

Primary human foreskin fibroblasts (HFF) were cultivated in MEM containing 5% (v/v) fetal calf serum. Infection analysis was restricted to cell passage numbers below twenty. Human cytomegalovirus strain AD169 (ATCC) was grown in HFF cells and quantitated for infectivity by the plaque reduction assay. Aliquots were stored at -80°C.

### Construction of recombinant cytomegalovirus

For construction of a recombination vector, two linker sequences were inserted into the pBlueScribe vector pBS+ (Stratagene): the first contained restriction sites for Nhel, Spel, Pacl and Bgll followed by a loxP sequence (ATAACTTCGTATAGCATACATTATACGAAGTTAT) and was introduced into Pstl/Xbal sites of the vector; the second contained another loxP sequence followed by restriction sites Hpal, Clal and Pmel and was introduced into BamHI/Asp718 sites. A gene cassette comprising of a "humanized" version of the ORF coding for GFP (gfp-h) under the control of the HCMV enhancer/promoter and the Ptk/PY441 enhancer-driven neoR selection marker was excised from plasmid pUF5 (Zolotukhin et al., 1996, J. Virol. 70, 4646-4654) and inserted into the recombination vector via Bglll sites.

At the 5' and 3'-positions of this loxP-flanked gene cassette, two HCMV sequences with homology to the gene region containing the open reading frames US9 and US10 were inserted. For this, viral sequences were amplified from template pCM49 (Fleckenstein et al., 1982, Gene 18, 39-46) via PCR in a 35-cycle program (denaturation 45 sec at 95°C, annealing 45 sec at 55°C and elongation 2 min at 72°C) by the use of Vent DNA polymerase (New England Biolabs). A US10-specific sequence of 1983 bp in length was generated using primers US10[200900]Spel (GCTC**ACTAGT**GGCCTAGCCTGGCTCATGGCC) and US10[198918]Pacl (GTCC**TTAATTAA**GACGTGGTTGTGGTCACCGAA) and inserted at the vector 5' cloning position via Spel/Pacl restriction sites (see bold-print). A US9-specific sequence of 2010 bp was generated using primers US9-3'Pmel (CTCG**GTTTAAA**CGACGTGAGGCGCTCCGTCACC) and US-5' Clal (TTGC**ATCGAT**ACGGTGTGAGATACCACGATG) inserted at the vector 3' cloning position via Pmel/Clal restriction sites.

The resulting construct pHM673 was linearized by the use of restriction enzyme Nhel and transfected into HEF cells via the electroporation method using a Gene Pulser (Bio-rad; 280 V, 960 µF, 400 Ω). After 24 h of cultivation, cells were used for infection with 1 PFU/ml of HCMV strain AD169. Selection with 200 µg/ml G418 was started 24 h post infection. Following 3 weeks of passage in the presence of G418, GFP fluorescence could be detected in most of the infected cells. Plaque assays were performed with infectuous culture supernatant on HFF cells and single virus plaques were grown by transfer to fresh HFF cells cultured in 48-well plates. DNA was isolated from cells of 32 fluorescence-positive wells and confirmed for the presence of recombinant virus by PCR. For this, primers US9[198789] (TGACGCGAGTATTACGTGTC) and US10[199100] (CTCCTCCTGATATGCGGTT) were used resulting in an amplification product of 312 bp for wild-type AD169 virus and approximately 3.5 kb for recombinant virus.

### Plaque assay

HFF cells were cultivated in 12-well plates to 90-100% confluency and used for infection with dilutions of virus-positive cell culture supernatants. Virus inoculation was performed for 90 min at 37°C under occasional shaking before virus was removed and the cell layers were rinsed with PBS. Overlays of MEM 5% (v/v) fetal calf serum and 0.3% (w/v) agarose were added to each well and all samples were incubated at 37°C in a 5% CO₂ atmosphere for approximately 12 days. Finally, overlays were removed and the formation of foci was visualized by staining with 1 % crystal violet in 20% ethanol for 1 min. After repeated rinsing with PBS, plates were air-dried at room temperature and plaque numbers were counted with a light microscope. For the recombinant AD169-GFP virus, quantification of plaque assays could also be performed without crystal violet staining by a direct counting of the amount of green fluorescent plaques using fluorescence microscopy.

### Antiviral compounds

The reference compounds used for antiviral studies, ganciclovir (GCV, Cymeven), foscarnet sodium (FOS, Foscavir) and cidofovir (CDV, Vistide) were purchased from Syntex Arzneimittel (Aachen, Germany), Sigma-Aldrich (Germany) and Pharmacia & Upjohn S.A. (Luxembourg), respectively. Stocks were prepared in aequeous solution and stored at -20°C. The test compounds were dissolved in DMSO and aliquots were stored at -20°C.

### GFP infection assay

HFF cells were cultivated in 12-well plates to 90-100% confluency and used for infection with 0,5xTCID₅₀ of AD169-GFP virus. Virus inoculation was performed for 90 min at 37°C with occasional shaking before virus was removed and the cell layers were rinsed with PBS. Infected cell layers were incubated with 2 ml of MEM containing 5% (v/v) fetal calf serum and optionally of the respective test substances or DMSO as control. Infected cells were incubated at 37°C in a 5% CO₂ atmosphere for 7 days and harvested by trypsination and centrifugation. 200 µl of lysis buffer (25 mM Tris pH 7.8, 2 mM DTT, 2 mM trans-1,2-diaminocyclohexane-N,N,N',N'-tetraacetic acid, 1% Triton X-100, 10% glycerol) was added to each cell pellet and lysis was achieved by incubation for 10 min at 37°C followed by a 30-min incubation at room temperature on a shaker. Lysates were centrifuged for 5 min at 15.000 rpm in an Eppendorf centrifugue to remove cell debris. Supernatants were transferred to an opaque 96-well plate for automated measuring of GFP signals in a Victor 1420 Multilabel Counter (Wallac). GFP units were converted to percent inhibition values relative to DMSO controls (set at 100% GFP expression).

### Indirect immunofluorescence analysis

Cells were either grown on Lab-Tek Permanox slides (Nunc) or harvested from 6-well plates, spotted onto glass slides with marked rings (Medco) and fixed by a 15-min treatment with 3% formaldehyde in PBS followed by permeabilization for 15 min in 0.1% Triton X-100 in PBS at room temperature. Blocking was achieved by incubation with Cohn Fraction II/III of human gamma-globulin (Sigma; 2 mg/ml) for 30 min at 37°C. The IE1/IE2-specific primary antibody MAb810 (Chemicon International, Inc. CA, USA; dilution 1:10.000) was incubated for 90 min, the secondary antibody (tetramethyl rhodamine [TRITC]-coupled anti-mouse antibody, Dianova, dilution 1:100) for 45 min at 37°C before analysis by fluorescence microscopy. In addition to indirect TRITC staining of IE1/IE2 proteins, GFP signals could be detected diretcly via the fluorescence isothiocyanate (FITC) channel. Nuclear counterstaining was carried out using Vectashield mounting medium including DAPI (Vector Laboratories, Burlingame, CA).

## Claims

1. Method for identifying compounds useful for treating and/or preventing Cytomegalovirus infection and/or associated diseases comprising:
a) contacting a test compound with one or more cellular kinases selected from the group consisting of RICK, RIP, NIK, MKK3, and SRPK-2; and
b) detecting a change in activity of said cellular kinase.

2. Method for detecting Cytomegalovirus infection and/or associated diseases in an individual comprising:
a) providing a sample from said individual; and
b) detecting activity, in said sample, of one or more cellular kinases selected from the group consisting of RICK, RIP, NIK, MKK3, and SRPK-2.

3. Method for detecting Cytomegalovirus infection and/or associated diseases in cells and/or cell lysates comprising:
a) providing a sample from said cells; and
b) detecting activity, in said sample, of one or more cellular kinases selected from the group consisting of RICK, RIP, NIK, MKK3, and SRPK-2.

4. A monoclonal or polyclonal antibody that binds to a cellular kinase selected from the group consisting of RICK, RIP, NIK, MKK3, and SRPK-2.

5. Method for preventing and/or treating Cytomegalovirus infection and/or associated diseases in an individual by administering a pharmaceutically effective amount of an inhibitor to said individual, wherein said inhibitor inhibits at least partially the activity of one or more cellular kinases selected from the group consisting of RICK, RIP, NIK, MKK3, and SRPK-2, or wherein said inhibitor inhibits at least partially the production of one or more cellular kinases selected from the group consisting of RICK, RIP, NIK, MKK3, and SRPK-2.

6. Method for regulating the production of Cytomegalovirus in an individual by administering an individual a pharmaceutically effective amount of an inhibitor wherein said inhibitor inhibits at least partially the activity of one or more cellular kinases selected from the group consisting of RICK, RIP, NIK, MKK3, and SRPK-2, or wherein said inhibitor at least partially inhibits the production of one or more cellular kinases selected from the group consisting of RICK, RIP, NIK, MKK3, and SRPK-2.

7. Method for regulating the production of Cytomegalovirus in cells by administering the cells a pharmaceutically effective amount of an inhibitor wherein said inhibitor inhibits at least partially the activity of one or more cellular kinases selected from the group consisting of RICK, RIP, NIK, MKK3, and SRPK-2, or wherein said inhibitor at least partially inhibits the production of one or more cellular kinases selected from the group consisting of RICK, RIP, NIK, MKK3, and SRPK-2 in the cells.

8. Method according to claim 5, 6, or 7 wherein the inhibitor is a monoclonal or polyclonal antibody which binds to a cellular kinase selected from the group consisting of RICK, RIP, NIK, MKK3, and SRPK-2.

9. Method for preventing and/or treating Cytomegalovirus infection and/or associated diseases in an individual by administering a pharmaceutically effective amount of an activator to said individual, wherein said activator activates at least partially the activity of one or more cellular kinases selected from the group consisting of RICK, RIP, NIK, MKK3, and SRPK-2, or wherein said activator activates or stimulates at least partially the production of one or more cellular kinases selected from the group consisting of RICK, RIP, NIK, MKK3, and SRPK-2.

10. Method for regulating the production of Cytomegalovirus in an individual by administering an individual a pharmaceutically effective amount of an activator wherein said activator activates at least partially the activity of one or more cellular kinases selected from the group consisting of RICK, RIP, NIK, MKK3, and SRPK-2, or wherein said activator at least partially activates or stimulates the production of one or more cellular kinases selected from the group consisting of RICK, RIP, NIK, MKK3, and SRPK-2.

11. Method for regulating the production of Cytomegalovirus in cells by administering the cells a pharmaceutically effective amount of an activator wherein said activator activates at least partially the activity of one or more cellular kinases selected from the group consisting of RICK, RIP, NIK, MKK3, and SRPK-2, or wherein said activator at least partially activates or stimulates the production of one or more cellular kinases selected from the group consisting of RICK, RIP, NIK, MKK3, and SRPK-2 in the cells.

12. Oligonucleotide that binds to the DNA or RNA encoding a cellular kinase selected from the group consisting of RICK, RIP, NIK, MKK3, and SRPK-2.

13. Method for regulating the expression of at least one cellular kinase selected from the group consisting of RICK, RIP, NIK, MKK3, and SRPK-2 in an individual comprising the step of administering the individual a pharmaceutically effective amount of an inhibitor wherein said inhibitor inhibits at least partially the transcription of DNA or the translation of RNA encoding one of said cellular kinases.

14. Method for regulating the expression of at least one cellular kinase selected from the group consisting of RICK, RIP, NIK, MKK3, and SRPK-2 in cells comprising the step of administering the cells a pharmaceutically effective amount of an inhibitor wherein said inhibitor inhibits at least partially the transcription of DNA or the translation of RNA encoding one of said cellular kinases.

15. Method according to claim 5, 6, 7, 13, or 14 wherein the inhibitor is a oligonucleotide which binds to the DNA and/or RNA encoding a cellular kinase selected from the group consisting of RICK, RIP, NIK, MKK3, and SRPK-2.

16. Method for regulating the expression of at least one cellular kinase selected from the group consisting of RICK, RIP, NIK, MKK3, and SRPK-2 in an individual comprising the step of administering the individual a pharmaceutically effective amount of an activator wherein said activator activates at least partially the transcription of DNA or the translation of RNA encoding one of said cellular kinases.

17. Method for regulating the expression of at least one cellular kinase selected from the group consisting of RICK, RIP, NIK, MKK3, and SRPK-2 in cells comprising the step of administering the cells a pharmaceutically effective amount of an activator wherein said activator activates at least partially the transcription of DNA or the translation of RNA encoding one of said cellular kinases.

18. A solid support useful for detecting Cytomegalovirus infection of an individual comprising one or more immobilized oligonucleotides, wherein said oligonucleotide(s) is (are) capable of detecting activity of one or more cellular kinases selected from the group consisting of: RICK, RIP, NIK, MKK3, and SRPK-2.

19. A solid support useful for detecting Cytomegalovirus infection of cells comprising one or more immobilized oligonucleotides, wherein said oligonucleotide(s) is (are) capable of detecting activity of one or more cellular kinases selected from the group consisting of RICK, RIP, NIK, MKK3, and SRPK-2.

20. A solid support useful for screening compounds useful for treating and/or preventing Cytomegalovirus infection comprising one or more immobilized oligonucleotides, wherein said oligonucleotide(s) encode one or more cellular kinases selected from the group consisting of RICK, RIP, NIK, MKK3, and SRPK-2.

21. A solid support useful for screening compounds useful for treating and/or preventing Cytomegalovirus infection comprising one or more immobilized cellular kinases selected from the group consisting of RICK, RIP, NIK, MKK3, and SRPK-2.

22. Composition useful to treat an individual afflicted with Cytomegalovirus and/or associated diseases comprising one or more inhibitors capable of inhibiting activity of one or more cellular kinases selected from the group consisting of RICK, RIP, NIK, MKK3, and SRPK-2 or capable of decreasing the expression of at least one cellular kinase selected from the group consisting of RICK, RIP, NIK, MKK3, and SRPK-2.

23. Composition useful to treat an individual afflicted with Cytomegalovirus and/or associated diseases comprising one or more activators capable of increasing activity of one or more cellular kinases selected from the group consisting of RICK, RIP, NIK, MKK3, and SRPK-2 or capable of increasing the expression of at least one cellular kinase selected from the group consisting of RICK, RIP, NIK, MKK3, and SRPK-2.

24. Composition useful to treat an individual afflicted with Cytomegalovirus comprising at least one compound selected from the group consisting of
6-(2,6-Dichlorophenyl)-8-methyl-2-(3-morpholin-4-yl-propylamino)-8H-pyrido[2,3-d]pyrimidin-7-one;
8-methyl-6-phenyl-2-(pyridin-4-yl-amino)-8H-pyrido[2,3-d]pyrimidin-7-one;
6-(2,6-Dichlorophenyl)-8-methyl-2-[3-(4-methylpiperazin-1-yl)-propylamino]-8H-pyrido[2,3-d]pyrimidin-7-one;
(3-Bromophenyl)-(6,7-dimethoxyquinazolin-4-yl)-amine;
(3-Bromophenyl)-(6,7-diethoxyquinazolin-4-yl)-amine;
2-(3,4-Dihydroxyphenyl)-3,5,7-trihydroxychromen-4-one;
5-Cloro-3-(1H-pyrrol-2-ylmethylene)-1,3-dihydroindol-2-one;
4-Quinolin-4-ylmethylene-4H-isoquinoline-1,3-dione
and/or phamaceutically acceptable salts of these compounds.

25. Composition according to any one of claims 17 - 19 further comprising pharmaceutically acceptable carriers, excipient, and/or diluents.

26. Use of the compounds selected from the group comprising:
6-(2,6-Dichlorophenyl)-8-methyl-2-(3-morpholin-4-yl-propylamino)-8H-pyrido[2,3-d]pyrimidin-7-one;
8-methyl-6-phenyl-2-(pyridin-4-yl-amino)-8H-pyrido[2,3-d]pyrimidin-7-one;
6-(2,6-Dichlorophenyl)-8-methyl-2-[3-(4-methylpiperazin-1-yl)-propylamino]-8H-pyrido[2,3-d]pyrimidin-7-one;
(3-Bromophenyl)-(6,7-dimethoxyquinazolin-4-yl)-amine;
(3-Bromophenyl)-(6,7-diethoxyquinazolin-4-yl)-amine and
pharmaceutically acceptable salts of these compounds as an inhibitor of the cellular kinase RICK.

27. Use of the compounds selected from the group comprising:
2-(3,4-Dihydroxyphenyl)-3,5,7-trihydroxychromen-4-one;
5-Cloro-3-(1 H-pyrrol-2-ylmethylene)-1,3-dihydroindol-2-one;
4-Quinolin-4-ylmethylene-4H-isoquinoline-1,3-dione; and
pharmaceutically acceptable salts of these compounds as an inhibitor of the cellular kinase RIP.

28. Use of a compound according to claim 26 or 27 for the manufacture of a pharmaceutical composition for prophylaxis and/or treatment of Cytomegalovirus infection and/or diseases associated therewith.
